# EUROPÄISCHE PATENTSCHRIFT

(11) **EP 1 587 624 B1**
(45) Veröffentlichungstag und Bekanntmachung des Hinweises auf die Patenterteilung: **14.12.2011**
(21) Anmeldenummer: 04702687.7
(22) Anmeldetag: 16.01.2004
(51) Int. Cl.: B01L 3/00, B01J 19/00

(54) **PROBENGEFÄSS FÜR ANALYSEN**
SAMPLE VESSEL FOR ANALYSES
RECIPIENT A ECHANTILLON POUR ANALYSES

(30) Priorität: 17.01.2003 DE 10302341; 10.05.2003 DE 10321042
(43) Veröffentlichungstag der Anmeldung: 26.10.2005
(73) Patentinhaber: Greiner Bio-One GmbH, 72636 Frickenhausen (DE)
(72) Erfinder: KNEBEL, Günther, 72622 Nürtingen (DE); STAPPERT, Jörg, 72138 Kirchentellinsfurt (DE); JEHLE, Heinrich, 72636 Linsenhofen (DE); KESSLER, Joachim, 89075 Ulm (DE)
(74) Vertreter: Schrell, Andreas
(86) Internationale Anmeldenummer: PCT/EP2004/000311
(87) Internationale Veröffentlichungsnummer: WO 2004/065009

(56) Entgegenhaltungen:
- WO-A-98/23957
- DE-A- 19 916 867

## Beschreibung

Die vorliegende Erfindung betrifft ein Probengefäß für Analysen, insbesondere einen Biochip-Träger, umfassend eine Trägerplatte mit mindestens einer Reaktionskammer, das zur medizinischen Diagnostik oder zur pharmazeutischen Wirkstoffsuche geeignet ist.

Mikroplatten für die Titration von biologischen oder chemischen Materialien beziehungsweise zur Durchführung von Fluoreszenz-, Lumineszenz- oder anderen Messungen sind bekannt. Mikroplatten, die als Multiküvetten ausgebildet sind, finden insbesondere in Forschung, Klinik und Industrie Verwendung, beispielsweise bei der Durchführung von Blutgruppenserologie, Antibiotika-Testreihen, Komplementtitrationen und anderen Laborarbeiten, bei denen beispielsweise geometrische Verdünnungsreihen erforderlich sind. Das allgemeine Standardformat für biochemische und zellbiologische Untersuchungen sind Mikrotiterplatten mit 96 Wells ("Probentöpfchen" oder Vertiefungen) mit einem Reaktionsvolumen von bis zu 500 µl pro Vertiefung. Zunehmend werden auch Mikrotiterplatten mit 384 Vertiefungen oder sogar mit 1536 Vertiefungen eingesetzt. Dieser Trend zur immer stärker werdenden Miniaturisierung wird vor allem durch die kombinatorische Chemie und das Hochdurchsatz-Screening, auch HTS (High Throughput Screening) genannt, forciert. Beide Bereiche gehören heute zu den Grundpfeilern der modernen pharmazeutischen Wirkstoffsuche.

Mittels HTS wird beispielsweise untersucht, ob sich in einer Substanzbibliothek ein Wirkstoff befindet, der als Basis für neue Medikamente eingesetzt werden kann. Die Komponenten der Substanzbibliothek werden dabei in einem Testverfahren bezüglich ihrer Reaktivität mit einem Target (Zielmolekül) untersucht. Die aufgefundenen Substanzen sind mögliche Kandidaten für einen Wirkstoff, der die Funktion des betreffenden Zielmoleküls beeinflussen kann. Die Detektion der Wirkstoffe erfolgt dabei entweder über optische Verfahren wie Absorption, Fluoreszenz, Lumineszenz oder über den Nachweis von Radioaktivität über Szintillation. Die Vielzahl der zu untersuchenden Wechselwirkungen bedingt eine große Varianz bei den Testsystemen und den damit verknüpften Detektionsarten.

Die Wirkstoffsuche erfordert, dass zunächst die Targets gefunden werden, die für das Entstehen von Krankheiten verantwortlich sind. Durch das wachsende Verständnis der modernen Molekularbiologie werden immer mehr krankheitsverursachende beziehungsweise krankheitsbeeinflussende Gene identifiziert, auf die dann mit geeigneten Medikamenten eingewirkt werden kann. Einen Meilenstein bei der Analyse von biologisch aktiven Molekülen, insbesondere zur Identifizierung der für das Entstehen von Krankheiten verantwortlichen Gene, stellen miniaturisierte Träger, sogenannte Biochips dar. An der Oberfläche solcher Träger können biologisch aktive Moleküle bekannter Zusammensetzung vollflächig oder in einem geordneten Raster immobilisiert oder synthetisiert werden. Bei den immobilisierten biologischen Molekülen kann es sich beispielsweise um Nucleinsäuren oder Fragmente davon beziehungsweise Proteine oder Fragmente davon handeln. Mit Hilfe von immobilisierte Nucleinsäuren aufweisenden Biochips, die auch als DNA-Arrays bezeichnet werden, kann beispielsweise die Nucleinsäure-Bestimmung in zu untersuchenden Proben wesentlich vereinfacht, beschleunigt, parallelisiert, automatisiert und präzisiert werden. DNA-Chips oder DNA-Arrays werden beispielsweise in der klinischen Diagnostik von Infektions-, Krebs- und Erbkrankheiten eingesetzt. Die Effizienz solcher DNA-Chips bei der Analyse von Proben beruht insbesondere darauf, dass nur noch geringe Probenvolumina benötigt werden und die Auswertung mittels hochempfindlicher Messverfahren erfolgen kann. Unter Verwendung solcher Chips lassen sich daher große Probenzahlen sehr schnell untersuchen. Auch Protein-Arrays sind bekannt, bei denen analog zu den DNA-Chips Proteine oder Peptide auf beispielsweise Kunststoffmembranen in einem geordneten und bekannten Raster angeordnet sind. Derartige Protein-Arrays werden vor allem zur Untersuchung der wechselseitigen Bindung von Proteinen, zum Beispiel Rezeptor-Ligand-Wechselwirkung, zur Identifizierung intrazellulärer Proteinkomplexe, zur Untersuchung von DNA-Protein-und RNA-Protein-Wechselwirkungen oder zur Analyse von Protein-Antikörper-Interaktionen eingesetzt. Mit Hilfe der Protein-Chip-Technologie konnten bereits zahlreiche Proteinmarker für Krebserkrankungen oder Krankheiten wie die Alzheimer-Demenz identifiziert werden.

Die Miniaturisierung der Mikroplatten-Testsysteme bringt zwar erhebliche Vorteile auf der Kostenseite, ist aber auch mit großen Problemen bei der technischen Realisierung verbunden. Aufgrund der Miniaturisierung der Mikrotiterplatten müssen auch die in den Wells durchzuführenden Tests immer stärker miniaturisiert werden. Dadurch bedingt werden auch an die Detektions-Vorrichtungen mit zunehmend kleineren Volumina erhöhte Anforderungen gestellt. So ist bekannt, dass bei den einzelnen Detektions-arten in extrem kleinen Volumina spezielle Probleme auftreten. Bei der Lumineszenzmessung bedeutet beispielsweise ein geringeres Probenvolumen auch ein geringeres Signal für die optische Detektion, was die Sensitivität der Messung beeinträchtigt. Die Absorptionsmessung wird vor allem durch den Meniskuseffekt der Flüssigkeitsoberfläche gestört, da der Meniskus in extrem kleinen Probenräumen sehr variabel verläuft. Einzig und allein die Fluoreszenzmessung unterliegt keiner Volumeneinschränkung. Allerdings ist hier die erreichbare Empfindlichkeit durch die Eigenfluoreszenz des Kunststoffmaterials der Mikrotiterplatten, die von den meisten Verfahren miterfasst wird, begrenzt.

Probleme ergeben sich insbesondere bei Mikroplatten mit mehreren hundert Wells. Da die Öffnung solcher Wells sehr klein und zudem häufig noch durch Ringwülste begrenzt ist, während das Volumen gleichzeitig relativ groß ist, lassen sich die zum Nachweis der Reaktionen in den Wells verwendeten Detektionssysteme, beispielsweise Scanner-Vorrichtungen, nicht senkrecht in einem optimalen Winkel von 90° über beziehungsweise in der Öffnung der Wells anordnen, sondern müssen schräg oberhalb beziehungsweise innerhalb der Öffnungen angeordnet werden, wobei der Winkel teilweise erheblich kleiner als 90° ist. Dadurch ergibt sich ein Abschattungseffekt, der die mittels der Detektionssysteme erfassten Analyse-Ergebnisse zusätzlich verfälscht. Auch für Spotter-Vorrichtungen, die dazu dienen, den Boden der Vertiefungen beispielsweise mit Nucleinsäuren zu beschichten, ergeben sich technische Probleme, da der Spotter relativ tief in die Wells eingeführt werden muss.

In der forschenden pharmazeutischen Industrie beziehungsweise in der Grundlagenforschung können die im Zusammenhang mit der Miniaturisierung stehenden Probleme bei Mikrotiterplatten häufig toleriert werden. Hier wird primär angestrebt, auf einer Platte sehr viele Proben in parallelen Ansätzen dem gleichen Testverfahren zu unterziehen. Daher ist es hier oftmals völlig ausreichend, den Unterschied der Signalintensität zwischen einzelnen Wells zu erfassen und somit eine mehr qualitative Aussage zu erhalten. In der klinischen Diagnostik ist die Situation jedoch vollkommen anders. Hier müssen beispielsweise sehr häufig Proben, zum Beispiel Körperflüssigkeiten, eines Patienten mehreren unterschiedlichen Testverfahren unter Verwendung unterschiedlicher Reaktanten unterzogen werden, wobei jeder Test vergleichsweise wenig Parallel-Ansätze umfassen kann. Umgekehrt ist es häufig auch notwendig, sehr viele Proben verschiedener Patienten auf einen einzigen Parameter zu testen. Im Gegensatz zum High Throughput-Screening müssen die einzelnen klinischen Tests allerdings aussagekräftige quantitative Aussagen ermöglichen, um beispielsweise den Ausbruch oder Verlauf einer Krankheit bei einem Einzelpatienten nachweisen zu können. Die bei den Detektions-Systemen in extrem kleinen Volumina auftretenden Probleme können daher in der klinischen Diagnostik zu gravierenden Fehlern der erhaltenen Messwerte führen. Die Genauigkeit der Detektion spielt daher in der klinischen Diagnostik eine erheblich größere Rolle als beispielsweise beim High Throughput-Screening von Wirkstoffen.

Das der vorliegenden Erfindung zugrunde liegende technische Problem besteht darin, ein Analysen-Probengefäß insbesondere für patientenspezifische klinisch-chemische Untersuchungen bereitzustellen, das nach Immobilisierung biologisch aktiver Moleküle insbesondere als Biochip eingesetzt werden kann und das die im Stand der Technik bekannten Nachteile überwindet und eine schnelle und zuverlässige Bestimmung klinischer Parameter erlaubt, wobei insbesondere eine quantitative fehlerfreie Detektion relevanter klinisch-chemischer Parameter unter Verwendung automatisierter Detektions-Technik möglich ist.

Die vorliegende Erfindung löst das ihr zugrunde liegende technische Problem durch die Bereitstellung eines Biochip-Trägers umfassend eine Trägerplatte mit mindestens einer dreidimensionalen Reaktionskammer, wobei die Reaktionskammer von einem Bodenteil und das nach oben hin geöffnete Volumen der Reaktionskammer seitwärts umfassenden Seitenwänden gebildet ist, wobei das Verhältnis zwischen der Fläche des Bodenteils und der Höhe der Seitenwände größer oder gleich 30, vorzugsweise größer als 50 ist. Dabei ist das Bodenteil und/oder mindestens eine Seitenwand der mindestens einen Reaktionskammer als Bindematrix mit einer funktionellen Gruppe, die die Bindung eines natürlichen oder synthetischen Moleküls, insbesondere eines biologisch aktiven Moleküls ermöglicht, ausgeführt. Weiterhin ist der mindestens einen Reaktionskammer eine Volumenvergrößerungskammer reversibel zugeordnet, wobei die Volumenvergrößerungskammer so auf den Biochip-Träger aufbringbar ist, dass eine flüssigkeitsdichte Verbindung zwischen den Seitenwänden der Reaktionskammer und den Seitenwänden der Volumenvergrößerungskammer gebildet wird.

Erfindungsgemäß ist insbesondere vorgesehen, dass das Verhältnis des Zahlenwertes der in mm² angegebenen Fläche des Bodenteils zu dem Zahlenwert der in mm angegebenen Höhe der Seitenwände, gemessen vom tiefsten Teil des, bevorzugt ebenen, Bodenteils zur Oberkante der Seitenwände mit bevorzugt jeweils gleicher Höhe, größer oder gleich 30, 35, 40, 45, 50, 60, 70, 80 oder 90 ist. Besonders vorteilhaft ist es, wenn das Verhältnis des Zahlenwertes der Bodenteil-Fläche zum Zahlenwert der Höhe der Seitenwände 30 bis 100, vorzugsweise 32 bis 80 beträgt. Besonders bevorzugt ist das Verhältnis der Größe der Bodenteil-Fläche zur Höhe der Seitenwände größer oder gleich 50. Erfindungsgemäß bevorzugt bildet die Oberkante der Seitenwände der Reaktionskammer den höchsten Teil des Biochip-Trägers.

Der erfindungsgemäße Biochip-Träger umfasst also eine Trägerplatte und mindestens eine dreidimensionale Reaktionskammer, wobei die Reaktionskammer von einem Bodenteil und von das Volumen der Reaktionskammer seitwärts umfassenden und abschließenden Seitenwänden gebildet wird, wobei die Reaktionskammer nach oben hin eine Öffnung, bevorzugt mit der gleichen Fläche und Geometrie wie das Bodenteil aufweist und wobei das Verhältnis zwischen der Fläche (F) des Bodenteils und der Höhe der Seitenwände, wie oben beschrieben ist, insbesondere größer oder gleich 30, vorzugsweise größer als 50 ist.

Die vorliegende Erfindung stellt also einen Biochip-Träger bereit, auf dem mindestens eine, in anderen bevorzugten Ausführungsformen jedoch mehrere Reaktionskammern oder Kavitäten angeordnet sind, wobei die Reaktionskammer oder die Reaktionskammern eine im Verhältnis zu ihrer Höhe relativ große Fläche des Bodenteils aufweisen. Bei den im Stand der Technik bekannten Mikroplatten-Systemen ist das Verhältnis zwischen der Fläche des Bodenteils der Kavität und ihrer Höhe kleiner als 5 und somit wesentlich kleiner als bei dem erfindungsgemäßen Biochip-Träger.

Das erfindungsgemäß erheblich vergrößerte Verhältnis zwischen der Fläche des Bodenteils der Reaktionskammer und der Höhe ihrer Seitenwände bietet einerseits den Vorteil, dass bei kleinem Volumen der Reaktionskammer eine große Oberfläche zur Verfügung steht, die mit sehr vielen Bindungsstellen zur Immobilisierung biologisch aktiver Moleküle versehen und somit zur Durchführung von Reaktionen genutzt werden kann. Andererseits erlaubt die erfindungsgemäße Reaktionskammer eine im Vergleich zu bekannten Mikrotiterplatten erheblich verbesserte Auswertung des in der Reaktionskammer durchgeführten Testes unter Verwendung herkömmlicher Detektions-Systeme, da die Messwerte verfälschende, durch die Abmessungen herkömmlicher Wells bedingte Fehlerquellen beseitigt sind. Aufgrund ihrer geringen Tiefe bietet die erfindungsgemäße Reaktionskammer beispielsweise die Möglichkeit, einen herkömmlichen Scanner oder eine andere herkömmliche Detektions-Vorrichtung senkrecht über beziehungsweise senkrecht innerhalb der Öffnung der Reaktionskammer in einem Winkel von 90° zu positionieren und dann die entsprechende Messung durchzuführen. Dadurch gibt es im Gegensatz zu bekannten Mikrotiterplatten keine Abschattungseffekte, die zu einer Verfälschung der erhaltenen Messwerte führen können. Aufgrund ihrer geringen Tiefe bieten die Reaktionskammern des erfindungsgemäßen Biochip-Trägers auch problemlos die Möglichkeit, beispielsweise eine Nucleinsäure unter Verwendung eines herkömmlichen Spotters auf das jeweilige Bodenteil der einzelnen Reaktionskammern aufzutragen, ohne dass der Spotter sehr tief in eine Reaktionskammer eingeführt werden muss.

Im Gegensatz zu herkömmlichen Biochips, bei denen biologische Moleküle in Form von Spots auf einen planaren ebenen Träger, insbesondere Glasträger, aufgetragen sind, und bei denen keine abgegrenzte Reaktionskammer oder getrennte Reaktionskammern vorliegen, weist der erfindungsgemäße Biochip-Träger eine Kompartimentierung in Form mindestens einer einzigen abgegrenzten Reaktionskammer oder in weiteren Ausführungsformen auch mehrerer oder vieler voneinander getrennter Reaktionskammern auf. Der Biochip-Träger lässt sich daher auch für Reaktionen mit ausschließlich in Lösung befindlichen Reaktanten einsetzen, wobei je nach Bedarf auch unterschiedliche Reaktionen mit unterschiedlichen Reaktanten durchgeführt werden können. Andererseits lässt sich der erfindungsgemäße Biochip-Träger auch zur Durchführung solcher Reaktionen einsetzen, bei denen mindestens ein Reaktant an der Oberfläche des Bodenteils der Reaktionskammer gebunden ist. Im Gegensatz zu herkömmlichen Biochips können aufgrund der Kompartimentierung des erfindungsgemäßen Biochip-Trägers bei Bedarf auch unterschiedliche Reaktionen mit gleichen oder unterschiedlichen gebundenen Reaktanten in den einzelnen Reaktionskammern des erfindungsgemäßen Biochip-Trägers durchgeführt werden. Dadurch, dass die Reaktionskammern des erfindungsgemäßen Biochip-Trägers als separate Kavitäten ausgeführt sind, lassen sich beispielsweise in den einzelnen Reaktionskammern eines erfindungsgemäßen Biochip-Trägers gleichzeitig unterschiedliche Nucleinsäure-Hybridisierungen mit unterschiedlichen Nucleinsäuren und/oder unterschiedliche Protein-Protein-Bindungsreaktionen mit unterschiedlichen Proteinen durchführen, ohne dass sich diese einzelnen Reaktionen stören. Im Gegensatz zum erfindungsgemäßen Biochip-Träger bietet ein herkömmlicher Biochip ohne Kompartimentierung diese Möglichkeit nicht. Der erfindungsgemäße Biochip-Träger vereinigt also in vorteilhafter Weise die Vorzüge der Chip-Technologie mit den Vorteilen der Mikrotiterplatten-Technologie.

Der erfindungsgemäße Biochip-Träger ist insbesondere zur Durchführung patientenspezifischer Nucleinsäure-Analysen oder Protein-Analysen geeignet. Erfindungsgemäß ist beispielsweise vorgesehen, dass die Bodenfläche der erfindungsgemäßen Reaktionskammer mit chemischen Gruppen funktionalisiert ist, die eine Bindung biologischen Molekülen, insbesondere von Nucleinsäure-Sonden mit bekannter Nucleotidsequenz oder mit Proteinen mit bekannter Aminosäuresequenz erlauben. Durch Immobilisierung biologisch aktiver Moleküle lässt sich unter Verwendung des erfindungsgemäßen Bio-chip-Trägers mit funktionalisierten Reaktionskammern also ein Bio-chip herstellen, der beispielsweise eine Vielzahl diagnostischer Nucleinsäure-Hybridisierungen oder Nucleinsäure-Protein-Bindungsreaktionen oder Protein-Protein-Bindungsreaktionen ermöglicht.

Erfindungsgemäß ist in einer Ausführungsform der Erfindung weiterhin vorgesehen, dass der erfindungsgemäße Biochip-Träger Abmessungen aufweist, die das Einsetzen des erfindungsgemäßen Bio-chip-Trägers in eine herkömmliche Mikrotiterplatte mit Abmessungen nach SBS (Society of Biomolecular Screening)-Standard erlauben. In einer Ausführungsform ist der erfindungsgemäße Biochip-Träger in Form eines Streifens ausgeführt, dessen Länge ein Einsetzen in eine Mikrotiterplatte nach SBS-Standard ermöglicht, während die Breite des Streifens variiert werden kann. Die Streifenform des erfindungsgemäßen Biochips ist insbesondere in der klinischen Diagnostik, beispielsweise bei der Erfassung klinisch-chemischer Parameter eines Einzelpatienten äußerst vorteilhaft. Auf einem Streifen können die Reaktionskammern linear hintereinander in Reihe angeordnet sein, wobei pro Streifen auch mehrere Reihen nebeneinander angeordnet sein können.

Bei der klinischen Diagnostik müssen beispielsweise verschiedene Proben, zum Beispiel verschiedene Körperflüssigkeiten, eines Einzelpatienten einer Vielzahl unterschiedlicher klinisch-chemischer Tests unterzogen werden. Diese unterschiedlichen Tests können sich sowohl bezüglich ihrer Verfahrensschritte als auch bezüglich der erforderlichen Bedingungen, beispielsweise hinsichtlich des benötigten Temperaturbereiches beziehungsweise -profiles, erheblich voneinander unterscheiden. Ein gemeinsames Merkmal dieser Tests besteht häufig nur darin, dass die gleiche Probe, beispielsweise eine Körperflüssigkeit, in einer begrenzten Anzahl von Paralleluntersuchungen analysiert werden soll. Der zum Beispiel als Streifen ausgeführte erfindungsgemäße Biochip-Träger kann, beispielsweise in Abhängigkeit von der Anzahl der durchzuführenden Tests, mindestens eine Reaktionskammer, vorzugsweise jedoch mehrere, beispielsweise 2, 3, 4, 5, 6, 7, 8 oder mehr Reaktionskammern aufweisen, zum Beispiel in reihenweiser Anordnung. Erfindungsgemäß kann zum Beispiel der in Streifenform ausgeführte erfindungsgemäße Biochip-Träger auch 2ⁿ Reaktionskammern mit n ≥ 0, zum Beispiel 2, 4, 8 oder 16 Reaktionskammern aufweisen. Diese Anzahl von Reaktionskammern auf einem erfindungsgemäßen Biochip-Träger reicht in aller Regel aus, um beispielsweise einen Einzelpatienten-spezifischen Test mit entsprechenden Kontrollen und Parallel-Ansätzen durchzuführen. Sofern in einer anderen bevorzugten Ausführungsform der Biochip-Träger im Format einer rechteckigen Mikrotiterplatte zum Beispiel im SBS-Standardformat ausgeführt ist, können insgesamt 1 bis zum Beispiel 1536, vorzugsweise 1, 12, 24, 36, 48, 96 oder weitere Vielfache von 8 oder 12 Reaktionskammern vorhanden sein, vorzugsweise in Matrixform. Der Biochip-Träger kann in Matrixform auch so ausgebildet sein, dass er Abmessungen aufweist, die ein Einsetzen in eine Mikrotiterplatte nach SBS-Standard ermöglicht. Dieser matrixförmige Biochipträger weist in bevorzugter Ausführungsform Längen- und Breitenabmessungen auf, die im Wesentlichen der einer Mikrotiterplatte entsprechen, so dass der Biochip-Träger, bevorzugt reversibel, auf oder teilweise in der herkömmlichen Mikrotiterplatte aufgebracht, aufgesteckt oder eingeclipst werden kann und diese vollständig oder im Wesentlichen vollständig nach oben hin bedeckt.

Unterschiedliche Einzelpatienten-spezifische Tests können in vorteilhafter Weise auf unterschiedlichen erfindungsgemäßen Biochip-Trägern beziehungsweise Biochips durchgeführt werden, die unter Verwendung unterschiedlicher erfindungsgemäßer Biochip-Träger hergestellt wurden. Die zur Durchführung unterschiedlicher klinisch-chemischer Tests verwendeten Biochip-Träger beziehungsweise Biochips können sich beispielsweise dadurch unterscheiden, dass in ihren Kavitäten unterschiedliche Nucleinsäuren oder Proteine immobilisiert sind. Die für die Diagnose bei einem Einzelpatienten verwendeten unterschiedlichen Biochip-Träger beziehungsweise Biochips zum Beispiel ausgeführt in Streifenform, die für unterschiedliche Tests eingesetzt werden sollen, können erfindungsgemäß in die gleiche Mikrotiterplatte eingesetzt werden, um unter Verwendung geeigneter Pipettiertechnik die gleiche Probe eines Einzelpatienten gleichzeitig auf alle Biochip-Träger beziehungsweise Biochips aufzutragen. Nach Auftragen der Probe dieses Einzelpatienten können die zur Durchführung spezifischer Tests verwendeten Biochip-Träger beziehungsweise Biochips mit entsprechenden Biochip-Trägern oder Biochips, die Proben anderer Einzelpatienten aufweisen, auf anderen Mikrotiterplatten kombiniert werden, so dass diese Biochips gemeinsam den gleichen Test-Verfahrensschritten oder den gleichen TestBedingungen unterworfen werden. Nach erfolgter Testdurchführung können die entsprechenden Einzelpatienten-spezifischen Biochip-Träger beziehungsweise Biochips wieder auf einer separaten Mikrotiterplatte kombiniert und gemeinsam unter Verwendung eines einzigen Detektions-Systems ausgewertet werden. Die Streifenform des erfindungsgemäßen Biochip-Trägers erlaubt somit eine hohe Flexibilität bei der Probenaufarbeitung und -auswertung.

Im Zusammenhang mit der vorliegenden Erfindung wird unter einem "Biochip" eine Vorrichtung verstanden, die einen Träger mit mindestens einer Kavität oder Reaktionskammer umfasst, in der biologisch aktive Moleküle, beispielsweise Nucleinsäuren oder Proteine, immobilisiert oder fixiert sind und mit deren Hilfe beispielsweise mittels Hybridisierungs- und/oder Bindungsverfahren, auch eine kleine Menge eines Liganden, der unter geeigneten Bedingungen an diese biologisch aktive Moleküle binden kann, auch in einer kleinen Probe nachgewiesen werden kann. Der Biochip kann als Chipmodul, Reaktionsmodul, Testmodul, Testvorrichtung, Analysenmodul, Analysenkammer oder Analysenvorrichtung eingesetzt werden.

Unter einem "Biochip-Träger" wird im Zusammenhang mit der vorliegenden Erfindung eine Vorrichtung verstanden, die eine Trägerplatte mit mindestens einer Kavität oder Reaktionskammer umfasst, in der biologisch aktive Moleküle wie Nucleinsäuren oder Proteine immobilisiert oder fixiert werden können. Der Biochip-Träger kann also zur Herstellung eines Biochips verwendet werden, indem biologisch aktive Moleküle in der Reaktionskammer, insbesondere an der funktionalisierten Oberfläche des Bodenteils der Reaktionskammer, immobilisiert oder fixiert werden.

Unter der "Trägerplatte" des Biochip-Trägers wird ein dünnes und ebenes Element mit vorzugsweise rechteckiger Form verstanden, das aus einem Metall, einem Metalloxid, einem Kunststoff, einer Membran, Glas, Keramik oder einem Hybrid beziehungsweise einer Kombination davon besteht. Im Zusammenhang mit der Erfindung bedeutet dies, dass die Trägerplatte des erfindungsgemäßen Biochip-Trägers vollständig aus einem der vorstehend genannten Materialien besteht oder dieses im Wesentlichen enthält oder vollständig aus einer Kombination dieser Materialien besteht oder diese im Wesentlichen enthält oder dass die Oberfläche der Trägerplatte des erfindungsgemäßen Biochip-Trägers vollständig aus einem der vorstehend genannten Materialien besteht oder diese im Wesentlichen enthält oder vollständig aus einer Kombination dieser Materialien besteht oder diese im Wesentlichen enthält. Die Trägerplatte oder ihre Oberfläche besteht dabei zumindest zu etwa 50 %, 60 %, vorzugsweise zu etwa 70 %, bevorzugt zu etwa 80 % und am bevorzugtesten zu etwa 100 % aus einem der vorstehend genannten Materialien oder einer Kombination solcher Materialien. In bevorzugter Ausführungsform besteht die Trägerplatte des erfindungsgemäßen Biochip-Trägers zu etwa 100 % aus Kunststoff. Die Trägerplatte ist Träger der mindestens einen Reaktionskammer und macht diese handhabbar, dass heißt fungiert als Rahmen oder Halterung. In bevorzugter Ausführungsform weist die Trägerplatte Auflageflächen und/oder Befestigungsvorrichtungen auf, zum Beispiel Einrastvorrichtungen, Steckvorrichtungen oder sonstige Vorrichtungen, die ein vorzugsweises reversibles Aufsetzen, Aufstecken, Verbinden oder Aufclipsen des Mikrochip-Trägers auf oder in eine herkömmliche Mikrotiterplatte erlauben, wobei der Biochip-Träger die Kavitäten der Mikrotiterplatte ganz oder teilweise nach oben abdeckt. Die mindestens eine Reaktionskammer kann auf oder in der Trägerfläche ausgebildet sein. Die mindestens eine Reaktionskammer kann also integral, dass heißt einstückig mit der Trägerplatte ausgeführt sein, sie kann in einer anderen Ausführung aber auch auf die Trägerplatte als separate Einheit aufgesetzt und reversibel oder irreversibel verbunden sein. Die mindestens eine Reaktionskammer kann demgemäß aus den Materialen bestehen oder diese umfassen, die für die Trägerplatte vorstehend beschrieben wurden.

Unter einer "Reaktionskammer" oder "Kavität" wird ein geometrischer Körper verstanden, der aus einem Bodenteil und das Bodenteil seitwärts umgrenzenden Seitenwänden besteht und eine Öffnung aufweist, die von der Oberkante der Seitenwände gebildet wird. Unter einer "Reaktionskammer" oder "Kavität" wird also ein räumliches Gebilde verstanden, dass aus einem Bodenteil und Seitenwänden besteht; Bodenteil und Seitenwände sind so zueinander angeordnet, dass sie ein Volumen nach unten und seitwärts hin umfassen, wobei das umfasste Volumen nach oben hin eine Öffnung aufweist. Die Seitenwände können zum Beispiel als Wall, Wand, Steg, Erhebung, Wulst oder Ringwulst ausgeführt sein. Die Reaktionskammer umschließt so nach unten und seitwärts mit Öffnung nach oben ein Volumen, innerhalb dessen biochemische Reaktionen, insbesondere Nucleinsäure-Hybridisierungen, DNA-Protein-Bindungsreaktionen, Protein-Protein-Bindungsreaktionen etc. durchgeführt werden können. Erfindungsgemäß ist vorgesehen, dass der Bodenteil der Reaktionskammer eine ebene, plane Fläche darstellt, wobei das Bodenteil der Reaktionskammer im Grundriss erfindungsgemäß insbesondere die Form eines Kreises, Rechtecks, Quadrates, Sechsecks, Polygons oder einer Ellipse aufweist. Vorzugsweise weisen alle Seitenwände einer Reaktionskammer die gleiche Höhe auf. Vorzugsweise stehen alle Seitenwände rechtwinklig auf dem Bodenteil, wobei jedoch auch davon abweichende Winkel zwischen Seitenwand und Bodenteil Ausführungsformen dieser Erfindung darstellen.

Eine besonders bevorzugte Ausführungsform der Erfindung betrifft einen Biochip-Träger, bei dem die Oberkante der Seitenwände der Reaktionskammer den höchsten Teil des erfindungsgemäßen Biochip-Trägers darstellt. Im Zusammenhang mit der vorliegenden Erfindung ist die "Oberkante der Reaktionskammer" der oberste, das heißt höchste Punkt der Reaktionskammer, der durch die Seitenwände der Reaktionskammer vorgegeben ist. Erfindungsgemäß ist vorgesehen, dass die Oberkanten von mindestens zwei, vorteilhafterweise allen Reaktionskammern eines Biochip-Trägers auf gleicher Höhe liegen.

Eine besonders bevorzugte Ausgestaltung der Erfindung betrifft einen Biochip-Träger, bei dem die Oberkante der Reaktionskammer in einer Ebene oberhalb der Oberkante der Trägerplatte des Biochip-Trägers liegt. Im Zusammenhang mit der vorliegenden Erfindung wird unter der "Oberkante der Trägerplatte" der nach oben, das heißt in die gleiche Richtung wie die Öffnung der Reaktionskammer hin orientierte oberste Bereich der Trägerplatte verstanden, insbesondere ein Bereich, der im Wesentlichen über die gleiche Höhe der gesamten Trägerplatte verläuft und auch als deren Oberfläche bezeichnet werden kann.

In einer bevorzugten Ausgestaltung wird das Bodenteil der Reaktionskammer durch die Trägerplatte gebildet, während die Seitenwände der Reaktionskammer zum Beispiel als Erhebungen auf der Trägerplatte ausgebildet sind, und in bevorzugter Ausführung im Grundriss zum Beispiel das Bodenteil quadratisch, polygonal, sechseckig, elliptisch, rechteckig oder kreisförmig umfassen. In bevorzugter Ausführungsform liegt das Bodenteil in einer Ebene mit der Oberfläche der Trägerplatte. Die zum Beispiel als Erhebungen ausgebildeten Seitenwände der Reaktionskammer können beispielsweise als Ringwulst beziehungsweise Ringwall vorliegen, das heißt die Oberkante der Reaktionskammer verläuft um den Umfang der Reaktionskammer und ist von dem Ringwulst beziehungsweise dem Ringwall der nächsten Reaktionskammer durch eine Vertiefung getrennt. Die Oberkante der Reaktionskammer stellt in dieser Ausführungsform dann den obersten Bereich des Ringwulstes dar.

In einer weiteren Ausführungsform der vorliegenden Erfindung gemäß der die Seitenwände durch Erhebungen gebildet werden und die Oberkante der Seitenwände oberhalb der Oberkante der Trägerfläche liegt, ist der Biochip-Träger derart ausgestaltet, dass die mindestens eine Reaktionskammer auf einem Sockel, Podest oder Erhebung auf der Trägerplatte angeordnet ist. Der Grundriss dieses Sockels entspricht in bevorzugter Ausführungsform dem Grundriss des Bodenteils der jeweiligen Reaktionskammer. Eine derartig ausgestaltete Ausführungsform umfasst daher eine Trägerplatte, auf der sich mindestens eine auf einem Sockel angeordnete Reaktionskammer befindet. Die vorzugsweise ebene, planare Fläche des Bodenteils befindet sich dementsprechend oberhalb der Oberkante der Trägerplatte und zwar in einer vertikalen Distanz, die der Höhe des Sockels entspricht. Bevorzugt ist vorgesehen, dass für jede Reaktionskammer des Biochip-Trägers ein eigener Sockel vorgesehen ist, so dass die Anzahl der Sockel der Anzahl der Reaktionskammern entspricht. Die Ausgestaltung der Reaktionskammer selbst, insbesondere das Verhältnis der Grundfläche des Bodenteils zu der Höhe der Seitenwände sowie die geometrische Ausgestaltung der Seitenwände des Grundrisses der Reaktionskammer etc; ist wie für die anderen Ausführungsformen vorstehend beschrieben.

Die Erfindung stellt in einer weiteren bevorzugten Ausführungsform einen Biochip-Träger bereit, wobei die Oberkante der Reaktionskammer und die Oberkante der Trägerplatte in der gleichen Ebene liegen und das Bodenteil unterhalb der Oberkante der Trägerplatte angeordnet ist. Das heißt, erfindungsgemäß ist vorgesehen, dass die Reaktionskammer innerhalb und/oder unterhalb der Trägerplatte als Vertiefung angeordnet ist, gleichsam also in die Trägerplatte abgesenkt ist, wobei die Öffnung der Reaktionskammer mit der Oberkante der Trägerplatte abschließt, ohne dass die Öffnung der Reaktionskammer von als Erhebung ausgeführten Seitenwänden, zum Beispiel einem Ringwulst oder einem Ringwall umgeben ist. Die Höhe der vertikalen Seitenwände entspricht in dieser Ausführung der Distanz zwischen, bevorzugt ebenem, Bodenteil zu der Oberkante der Trägerplatte. Die Seitenwände werden in dieser Ausführungsform von der Trägerplatte gebildet.

Selbstverständlich kann die Reaktionskammer auch nur teilweise in die Trägerplatte abgesenkt sein, so dass das Bodenteil zwar unterhalb der Oberkante der Trägerplatte liegt, der untere Teil der Seitenwände durch die Trägerplatte gebildet wird und oberhalb der Trägerplatte sich die Seitenwände in Verlängerung der Seitenwandanteile aus der Vertiefung über die Oberkante der Vertiefung hinaus erstrecken. Die Oberkante der Seitenwand liegt dann oberhalb der Oberkante der Trägerplatte, so dass auch in dieser Ausführung die Seitenwände als Erhebungen ausgeführt sind.

In bevorzugter Ausführungsform der Erfindung weist der erfindungsgemäße Biochip-Träger wie bereits erläutert Abmessungen auf, die das passgenaue Einsetzen des Biochip-Trägers in eine Mikrotiterplatte mit Abmessungen gemäß SBS (Society of Biomolecular Screening)-Standard erlauben. Wenn der Biochip-Träger exakt oder im Wesentlichen exakt die Abmessungen einer Mikrotiterplatte nach SBS-Standard aufweist, ist erfindungsgemäß vorgesehen, dass die Anzahl von Reaktionskammern auf der Trägerplatte mindestens 1, 4, 8, 12 oder ganzzahlige Vielfache von 8 oder 12 davon beträgt. Erfindungsgemäß bevorzugt ist vorgesehen, dass der erfindungsgemäße Biochip-Träger als Streifen ausgeführt ist. In einer besonders bevorzugten Ausgestaltung der Erfindung weist der erfindungsgemäße Biochip-Träger Abmessungen von etwa 75 x 25 mm (Länge x Breite, Länge im folgenden: größere Abmessung in der Ebene der Matrix oder des Streifens, Breite im folgenden: kleinere Abmessung in der genannten Ebene) auf. Wenn der erfindungsgemäße Biochip-Träger diese Abmessungen aufweist, ist erfindungsgemäß vorgesehen, dass auf der Trägerplatte mindestens eine, aber auch zwei parallele Reihen von Reaktionskammern angeordnet sind, wobei jede Reihe jeweils mindestens eine, aber auch zwei oder mehrere, zum Beispiel acht hintereinander angeordneten beabstandete Reaktionskammern umfasst.

In einer weiteren bevorzugten Ausführungsform der Erfindung ist vorgesehen, dass der in Streifenform vorliegende erfindungsgemäße Biochip-Träger eine Breite von 9 mm aufweist. Die Länge des Streifens ist bevorzugt so gewählt, dass sie das Einsetzen des Biochip-Trägers in eine Standard-Mikrotiterplatte (SBS) ermöglicht, vorzugsweise so, dass die Länge des Streifens rechtwinklig zur Länge der Standard-Mikrotiterplatte angeordnet ist. Erfindungsgemäß ist vorgesehen, dass auf dem erfindungsgemäßen Biochip-Träger, der diese Abmessung aufweist, eine Reihe von acht hintereinander angeordneten beabstandeten Reaktionskammem angeordnet ist.

In einer besonders bevorzugten Ausführungsform der Erfindung weisen die auf dem erfindungsgemäß in Streifenform ausgeführten Biochip-Träger, insbesondere auf dem Biochip-Träger mit den Abmessungen von etwa 75 x 25 mm und dem Biochip-Träger mit einer Breite von 9 mm, angeordneten Reaktionskammern eine kreisförmige Bodenfläche mit einem Durchmesser von 6 mm und eine Seitenwand-Höhe von 0,5 mm auf. Das Verhältnis des Zahlenwertes der Bodenteil-Fläche zum Zahlenwert der Höhe der Seitenwände beträgt in dieser Ausführungsform der Reaktionskammer etwa 56. In einer weiteren besonders bevorzugten Ausführungsform der Erfindung weisen die auf den in Streifenform ausgeführten Biochip-Träger angeordneten Reaktionskammern eine quadratische Bodenfläche mit einer Seitenlänge von 6 mm und eine Seitenwand-Höhe von 0,5 mm auf. In dieser Ausführungsform beträgt das Verhältnis des Zahlenwertes der Bodenteil-Fläche zum Zahlenwert der Höhe der Seitenwände 72.

Erfindungsgemäß ist in einer bevorzugten Ausführungsform vorgesehen, dass die Trägerplatte und die Reaktionskammern des erfindungsgemäßen Biochip-Trägers einstückig ausgeführt sind, wobei die Reaktionskammern ein integraler Bestandteil der Trägerplatte sind. Vorzugsweise besteht der Biochip-Träger aus Kunststoff. Besonders bevorzugt handelt es sich bei dem zur Herstellung des Biochips verwendeten Kunststoff um Cycloolefin-Copolymer (COC), Cycloolefinpolymer (COP), Acrylbutadienstyrol, Polyamid, Polycarbonat, Polyester, Polymethylmethacrylat, Polypropylen, Polystyrol, SMA (Styrol-Maleinsäureanhydrid-Copolymer) oder Styrolacrylnitril.

Erfindungsgemäß ist vorgesehen, dass das Bodenteil der mindestens einen Reaktionskammer als Bindematrix mit einer funktionellen Gruppe, die die Bindung eines natürlichen oder synthetischen Moleküls, insbesondere eines biologisch aktiven Moleküls ermöglicht, ausgeführt ist. In bevorzugter Ausführungsform besteht das als Bindematrix ausgeführte Bodenteil der Reaktionskammer aus Glas, einem Polymer, einer Membran oder einem Hybrid davon, wobei an der Oberfläche des Materials mindestens eine funktionelle Gruppe enthalten ist, die beispielsweise durch kovalente Bindung oder auch durch Brönstedt Säure-Base-Wechselwirkung ein natürliches oder synthetisches Molekül, insbesondere ein biologisch aktives Molekül an der Oberfläche des Bodenteils immobilisieren kann. Das Bodenteil der Reaktionskammer ist also erfindungsgemäß eine Bindematrix mit einer funktionellen chemischen Gruppe, die die Bindung, insbesondere kovalente Bindung, an eine komplementäre Gruppe eines natürlichen oder synthetischen Moleküls, insbesondere eines biologisch aktiven Moleküls ermöglicht. Im Zusammenhang mit der vorliegenden Erfindung wird daher unter einer ,,funktionellen Gruppe" eine auf dem Bodenteil der Reaktionskammer aufgebrachte chemische Gruppe verstanden, die in der Lage ist, mit einem zu immobilisierenden natürlichen oder synthetischen Molekül, insbesondere einem biologisch aktiven Molekül, derart zu interagieren, dass insbesondere eine kovalente oder ionische Bindung zwischen den beiden Bindungspartnern stattfinden kann.

In bevorzugter Ausführungsform besteht das Bodenteil der Reaktionskammer aus einem Polymer, das an der Oberfläche mindestens eine funktionelle Gruppe aufweist

Die Funktionalisierung der Oberfläche, insbesondere der Oberfläche eines Polymers, kann erfindungsgemäß unter Verwendung chemischer Funktionalisierungsverfahren, Plasmafunktionalisierungsverfahren, UV-Funktionalisierungsverfahren oder unter Verwendung von Graft-Polymeren erfolgen.

Eine bevorzugte Ausführungsform der Erfindung betrifft daher einen Biochip-Träger, wobei die funktionelle Gruppe unter Verwendung eines chemischen Funktionalisierungsverfahrens auf die Polymer-Oberfläche aufgebracht ist.

Sofern die Bodenfläche der Reaktionskammer aus einem Polymer mit aromatischen Resten, beispielsweise Phenyl-Gruppen, wie im Fall von Polystyrol, besteht, kann ein Chloromethyl-Spacer mittels 1-Chloro-Dimethylether in Anwesenheit einer Lewis-Säure, beispielsweise AlCl₃, über eine elektrophile aromatische Substitution des Aromaten auf das Polymer übertragen werden. Die resultierende Chloromethyl-Funktion ist gegenüber nucleophilen Reagenzien zur Knüpfung von Kohlenstoff-Element-Bindungen unter Chloridabspaltung aktiviert. Die Chloromethyl-Funktion kann dann weiter substituiert werden, wobei die Substitution durch N-Nucleophile, beispielsweise NH₃, oder O-Nucleophile, erfolgen kann. Durch die Wahl eines geeigneten Nucleophilen, wie zum Beispiel HO-Aryl-CHO oder Formylessigsäure HO(O)C-CH₂-CHO kann dann direkt eine Aldehyd-Funktion eingeführt werden. Eine weitere Möglichkeit zur kovalenten Bindung ist durch die Amid-Bindung gegeben. Die dafür benötigte oberflächenfixierte Carbonsäure kann durch Reaktion von Dicarbonsäuren mit Chloromethyl-funktionalisiertem Polystyrol gewonnen werden. Dabei wird eine Säurefunktion auf der Oberfläche fixiert, während die zweite zur Bindung mit dem Zielmolekül, also dem zu bindenden biologischen Molekül genutzt werden kann. Die verbleibende C(O)OH-Funktion kann dann durch Überführung in das entsprechende Säurechlorid aktiviert werden. Problematisch hierbei ist jedoch die instabile Funktion, die in situ sofort weiter umgesetzt werden sollte. Die kovalente Bindung der Säure-Funktion an das Amin ist jedoch auch ohne vorherige Aktivierung möglich.

Eine bevorzugte Ausgestaltung der Erfindung betrifft daher einen Biochip-Träger, wobei das Polymer des Bodenteils ein Polymer mit aromatischen Resten ist, an dessen Oberfläche Chloromethyl-Gruppen angelagert sind. Bei dem Polymer mit aromatischen Resten handelt es sich vorzugsweise um Polystyrol.

Eine weitere bevorzugte Ausgestaltung der Erfindung betrifft einen Biochip-Träger, bei dem das Bodenteil der Reaktionskammer aus einem Polymer besteht, das an der Oberfläche mindestens eine derivatisierte Chloromethyl-Funktion aufweist, wobei die Chloromethyl-Gruppe durch eine Aldehyd-Gruppe, eine Amin-Funktion oder durch eine Carbonsäure substituiert ist.

Bei einem gesättigten Polymer, beispielsweise TOPAS (Cycloolefin-Copolymer, Grundgerüst Norbornen; Hersteller Ticona Frankfurt, Deutschland), ist die Einführung einer CH₂Cl-Funktion nicht möglich, da keine aromatischen Liganden vorliegen. Gesättigte Polymere können jedoch über eine radikalische Chlorierung unter Verwendung eines Katalysators chloriert werden. Die so erhaltenen Kohlenstoff-Chlor-Einheiten lassen sich ebenfalls derivatisieren.

Eine bevorzugte Ausgestaltung der Erfindung betrifft daher einen Biochip-Träger, bei dem das Bodenteil der Reaktionskammer aus einem gesättigten Polymer besteht, dessen Oberfläche chloriert ist. Vorzugsweise handelt es sich bei dem gesättigten chlorierten Polymer um chloriertes TOPAS.

Eine weitere bevorzugte Ausführungsform der Erfindung betrifft einen Biochip-Träger, bei dem die funktionelle Gruppe unter Verwendung eines Plasma-Funktionalisierungsverfahrens auf die Oberfläche des das Bodenteil der Reaktionskammer bildenden Polymers aufgebracht ist.

Erfindungsgemäß ist also ebenfalls vorgesehen, eine Oberflächenfunktionalisierung unter Verwendung eines Plasma-Verfahrens einzuführen. Luftsauerstoffplasma erzeugt Radikale und verschiedenartigste Ionen, wobei auf der Polymeroberfläche insbesondere Sauerstoff-Funktionalitäten gebildet werden. Dabei werden aufgrund der hohen Anregungsenergie und der sehr harschen Bedingungen Kohlenstoff-Bindungen auf der Polymeroberfläche gebrochen und unterschiedliche Kohlenstoff-Sauerstoff-Funktionalitäten gebildet. Während C-O-Einfachbindungen gegenüber kovalenten Bindungen inaktiv sind, sind Carbonsäure-Funktionen gegenüber einer kovalenten Amid-Bildung aktiv. Aldehyde können aminomodifizierte Biomoleküle unter Ausbildung einer Schiff-Base immobilisieren.

Durch eine geeignete Modifikation der Reaktionsbedingungen können mittels Plasmafunktionalisierungen Amin-Funktionen auf der Polymer-Oberfläche ausgebildet werden. Die so erzeugten Amin-Funktionen können bereits zur kovalenten Bindung von CarboxylGruppen eingesetzt werden. Dabei gibt es zwei Reaktionsmöglichkeiten, nämlich die Amid-Bindung von terminalen -C(O)OH-Säurefunktionen und die Imin-Bildung aus den vorhandenen internen C=O-Doppelbindungen. Plasmabehandelte Polymeroberflächen lassen sich darüber hinaus auch problemlos derivatisieren. Beispielsweise können aminierte Oberflächen zu Aldehyd-Funktionen aufweisenden Oberflächen umfunktionalisiert werden, wobei gleichzeitig auch der Abstand zwischen der funktionellen Gruppe und der Polymer-Oberfläche vergrößert werden kann. Sauerstoff-haltige Oberflächen können einer Carbonsäure-Derivatisierung und anschließend einem Spacer-Einbau unter Erhalt der Aldehyd-Funktion unterworfen werden.

Die vorliegende Erfindung betrifft daher auch unter Verwendung von Plasmaverfahren erhaltene Biochip-Träger, wobei die Oberfläche Carbonsäure-, Aldehyd/Keton-, Amin-, Epoxy- und/oder Halogen-Funktionen aufweist.

Eine weitere bevorzugte Ausgestaltung betrifft Biochip-Träger, wobei die funktionelle Gruppe unter Verwendung von photoinduzierter Oberflächenfunktionalisierung auf die Oberfläche des Polymers aufgebracht ist, das das Bodenteil der Reaktionskammer bildet.

Zum Aufbringen funktioneller Gruppen auf die Polymer-Oberfläche ist auch das Verfahren der photoinduzierten Oberflächenfunktionalisierung geeignet. Dazu kann insbesondere das Anthrachinon-Verfahren eingesetzt werden. Dabei wird die für die kovalente Bindung des biologischen Moleküls benötigte funktionelle Gruppe durch eine chemische Verknüpfungsreaktion an ein Anthrachinon-Derivat gebunden. Für diesen Kopplungsschritt sind jedoch starke Donoren, beispielsweise OH, OMe oder SO₃H, am Anthrachinon-Gerüst erforderlich. Weitere Möglichkeiten zur Antrachinon-Derivatisierung ergeben sich aus der Verwendung von Aldrich 16,554-9 (C₁₄H₉O₂N), Aldrich 43,425-6 oder Aldrich 25,272-7. Anschließend wird die gesamte Einheit über einen radikalischen Mechanismus am Polymergerüst photochemisch fixiert.

Ein weiteres photoinduziertes Oberflächen-FunktionalisierungsVerfahren ist das photochemische Graften, wobei variable Basismaterialien durch kontrollierte reaktive Beschichtungen mit Polymeren, die unterschiedliche funktionelle Gruppen tragen können, realisiert werden. Die Initiierung der Reaktion erfolgt photochemisch, insbesondere unter Verwendung von UV-Licht.

Die vorliegende Erfindung betrifft daher auch einen-Biochip-Träger, wobei die funktionelle Gruppe durch Umsetzung von Anthrachinon mit einem Donor und anschließende photochemische Fixierung auf die Polymer-Oberfläche aufgebracht ist. Die vorliegende Erfindung betrifft ebenfalls einen Biochip-Träger, wobei die funktionelle Gruppe durch photochemisches Graften auf die Polymer-Oberfläche aufgebracht ist.

Eine weitere bevorzugte Ausgestaltung der Erfindung betrifft einen Biochip-Träger, wobei die funktionelle Gruppe unter Verwendung von Propfpolymerisation auf die Oberfläche des Polymers aufgebracht ist, das den Bodenteil der Reaktionskammer bildet.

Unpolare Matrixpolymere, beispielsweise Polypropylen, Polyester oder EPM (Ethylen-Propylen-monomer-Elastomer) können auch durch Aufpfropfen monomerer Substanzen mit polaren Gruppen unter Verwendung eines Zweischnecken-Kneters funktionalisiert werden. Derartige Pfropfpolymerisate lassen sich als Ausgangskomponenten zur Herstellung von Polymerblends und Polymerlegierungen einsetzen. Auf diese Weise können beispielsweise Polypropylen/Polyamid-Legierungen oder thermoplastische Elastomere (pp/EP(D)M-Blends; Polypropylen/EPM-Blend) unter Einbeziehung einer dynamischen Vernetzung der Elastomerphase erzeugt werden.

Eine weitere besonders bevorzugte Ausführungsform der Erfindung betrifft einen Biochip-Träger beziehungsweise einen unter Verwendung des erfindungsgemäßen Biochip-Trägers hergestellten Biochip, wobei am Bodenteil der Reaktionskammer ein natürliches oder synthetisches Molekül, insbesondere ein Biomolekül beziehungsweise ein biologisch aktives Molekül, gebunden ist. Im Zusammenhang mit der vorliegenden Erfindung handelt es sich bei einem "natürlichen Molekül" um ein Molekül, das vorzugsweise aus einer natürlichen Quelle oder einem natürlichen Material isoliert und nach Isolierung gegebenenfalls einer oder mehreren Abwandlungen, Modifikationen und/oder chemischen Derivatisierungen unterworfen wurde. Unter einem "synthetischen Molekül" wird insbesondere ein auf synthetischem Weg hergestelltes Molekül verstanden. Vorzugsweise weist das natürliche oder synthetische Molekül eine biologische Aktivität auf, das heißt ist ein biologisch aktives Molekül. Sofern das natürliche oder synthetische Molekül selbst keine biologische Aktivität aufweist, hat es zumindest die Fähigkeit, ein biologisch aktives Molekül zu binden oder damit ein Aggregat zu bilden und das biologisch aktive Molekül somit auf der Oberfläche des Bodenteils zu immobilisieren.

Bei dem Biomolekül beziehungsweise dem biologisch aktiven Molekül handelt es sich insbesondere um ein Protein, eine Nucleinsäure oder ein PNA-Molekül.

Erfindungsgemäß ist insbesondere vorgesehen, dass das biologisch aktive Molekül oder Biomolekül unter Erhalt seiner biologischen Aktivität an der Bodenfläche der Reaktionskammer gebunden oder immobilisiert ist oder gebunden oder immobilisiert werden kann. Unter der "biologischen Aktivität" des biologisch aktiven Moleküls oder Biomoleküls werden alle Funktionen verstanden, die dieses in einem Organismus in seiner natürlichen zellulären Umgebung ausübt. Wenn das Molekül ein Protein ist, kann es sich um spezifische katalytische oder enzymatische Funktionen, Funktionen in der Immunabwehr, Transport- und Speicherfunktion, Regulationsfunktion, Transkriptions- und Translationsfunktionen und dergleichen handeln. Handelt es sich bei dem biologischen Molekül um eine Nucleinsäure, kann die biologische Funktion beispielsweise in der Codierung eines Genproduktes bestehen oder darin, dass die Nucleinsäure als Matrize zur Synthese weiterer Nucleinsäuremoleküle oder als Bindungsmotiv für regulatorische Proteine verwendbar ist. "Beibehaltung der biologischen Aktivität" bedeutet, dass ein biologisch aktives Molekül nach Immobilisierung oder Bindung an der Polymer-Oberfläche, das heißt am Bodenteil der Reaktionskammer des erfindungsgemäßen Biochips-Trägers, die gleichen oder nahezu gleichen biologischen Funktionen in zumindest ähnlichem Ausmaß ausüben kann wie das gleiche Molekül in nicht-immobilisiertem Zustand unter geeigneten in vitro-Bedingungen beziehungsweise das gleiche Molekül in seiner natürlichen zellulären Umgebung. Der Begriff "Immobilisierung" bedeutet im Zusammenhang mit der vorliegenden Erfindung, dass ein Molekül so an die funktionellen Gruppen der Polymer-Oberfläche gebunden wird beziehungsweise ist, dass beispielsweise die dreidimensionale Struktur der für die biologische Aktivität erforderlichen Domäne(n) gegenüber dem nicht-immobilisierten Zustand nicht verändert ist und dass diese Domäne(n), beispielsweise Bindungstaschen für zelluläre Reaktionspartner, bei Kontakt mit anderen nativen zellulären Reaktionspartnern für diese frei zugänglich ist/sind.

Erfindungsgemäß ist vorgesehen, dass das an den erfindungsgemäßen Biochip-Träger, insbesondere am Bodenteil der Reaktionskammer, immobilisierte oder immobilisierbare biologische Molekül insbesondere eine Nucleinsäure, ein Protein, ein PNA-Molekül, ein Fragment davon oder ein Gemisch davon ist.

Im Zusammenhang mit der vorliegenden Erfindung wird unter einer "Nucleinsäure" ein Molekül verstanden, dass aus mindestens zwei über eine Phosphordiesterbindung verbundenen Nucleotiden besteht. Bei Nucleinsäuren kann es sich sowohl um eine Desoxyribonucleinsäure als auch eine Ribonucleinsäure handeln. Die Nucleinsäure kann sowohl einsträngig als auch zweisträngig vorliegen. Im Kontext der vorliegenden Erfindung kann eine Nucleinsäure also auch ein Oligonucleotid sein. Die an der Bodenfläche der Reaktionskammer des erfindungsgemäßen Biochip-Trägers gebundene Nucleinsäure weist vorzugsweise eine Länge von mindestens 10 Basen auf. Erfindungsgemäß kann die gebundene Nucleinsäure natürlichen oder synthetischen Ursprungs sein. Die Nucleinsäure kann erfindungsgemäß auch durch gentechnische Verfahren gegenüber der Wildtyp-Nucleinsäure modifiziert sein und/oder unnatürliche und/oder ungewöhnliche Nucleinsäure-Bausteine enthalten. Die Nucleinsäure kann mit Molekülen anderer Art, beispielsweise mit Proteinen, verbunden sein.

Im Zusammenhang mit der vorliegenden Erfindung wird unter einem "Protein" ein Molekül verstanden, das mindestens zwei über eine Amidbindung miteinander verbundenen Aminosäuren umfasst. Im Kontext der vorliegenden Erfindung kann ein Protein also auch ein Peptid, zum Beispiel ein Oligopeptid, ein Polypeptid oder zum Beispiel eine isolierte Protein-Domäne sein. Ein derartiges Protein kann natürlichen oder synthetischen Ursprungs sein. Das Protein kann durch gentechnische Verfahren gegenüber dem Wildtyp-Protein modifiziert sein und/oder unnatürliche und/oder ungewöhnliche Aminosäuren enthalten. Das Protein kann gegenüber der Wildtyp-Form derivatisiert sein, beispielsweise Glykosylierungen auf-weisen, es kann verkürzt sein, es kann mit anderen Proteinen fusioniert sein oder mit Molekülen anderer Art, beispielsweise mit Kohlenhydraten, verbunden sein. Erfindungsgemäß kann ein Protein, insbesondere ein Enzym, ein Rezeptor, ein Cytokin, ein Antigen oder ein Antikörper sein.

"Antikörper" bedeutet ein Polypeptid, das im Wesentlichen von einem oder mehreren Immunglubolin-Genen codiert wird, oder Fragmente davon, das/die einen Analyten (Antigen) spezifisch bindet/binden und erkennt/erkennen. Antikörper kommen beispielsweise als intakte Immunglobuline oder als eine Reihe von Fragmenten vor, die mittels Spaltung mit verschiedenen Peptidasen erzeugt werden. "Antikörper" bedeutet auch modifizierte Antikörper, beispielsweise oligomere, reduzierte, oxidierte und markierte Antikörper. "Antikörper" umfasst auch Antikörper-Fragmente, die entweder mittels Modifikation ganzer Antikörper oder mittels de novo-Synthese unter Verwendung von DNA-Rekombinationstechniken erzeugt worden sind.

Der Begriff "Antikörper" umfasst sowohl intakte Moleküle als auch Fragmente davon, wie Fab, F(ab)')₂ und Fv, die Epitop-Determinanten binden können.

Bei PNA (Peptide Nucleic Acid oder Polyamide Nucleic Acid)-Molekülen handelt es sich um Moleküle, die nicht negativ geladen sind und in gleicher Weise wie DNA wirken (Nielsen et al., 1991, Science, 254, 1497-1500; Nielsen et al., 1997, Biochemistry, 36, 5072-5077; Weiler et al., 1997, Nuc. Acids Res., 25, 2792-2799). PNA-Sequenzen umfassen ein Polyamid-Grundgerüst aus N-(2-Aminoethyl)-glycin-Einheiten und besitzen keine Glucose-Einheiten und keine Phosphat-Gruppen.

In einer weiteren Ausführungsform der Erfindung ist vorgesehen, dass das an den erfindungsgemäßen Biochip-Träger beziehungsweise an den unter Verwendung des erfindungsgemäßen Biochip-Trägers hergestellten Biochip, insbesondere an das Bodenteil der Reaktionskammer, immobilisierte oder gebundene biologische Molekülmarkierungen aufweist, die eine einfache Detektion dieser Moleküle unter Verwendung geeigneter Nachweisverfahren ermöglichen. Bei diesen Markierungen kann es sich beispielsweise um eine Fluoreszenzmarkierung, eine UV/VIS-Markierung, eine superparamagnetische Funktion, eine ferromagnetische Funktion und/oder eine radioaktive Markierung handeln. Als Nachweisverfahren für diese Markierung kommen beispielsweise Fluoreszenz- oder UV-VIS-Spektroskopie, Wellenleiter-Spektroskopie-, Impedanz-Spektroskopie-, elektrische und/oder radiometrische Verfahren in Betracht.

In einer Ausführungsform der Erfindung ist vorgesehen, dass in jeder Reaktionskammer des erfindungsgemäßen Biochip-Trägers beziehungsweise Biochips das gleiche biologisch aktive Molekül immobilisiert ist. In einer weiteren Ausführungsform der Erfindung ist vorgesehen, dass in jeder Reaktionskammer des erfindungsgemäßen Biochip-Trägers beziehungsweise Biochips ein anderes Molekül immobilisiert ist.

Die Erfindung betrifft einen wie vorstehend beschriebenen Biochip-Träger beziehungsweise Biochip, wobei dieser eine reversibel anbringbare, beispielsweise aufsteckbare und einer Volumenvergrößerungsvorrichtung zugeordnete, insbesondere mindestens eine nach oben und unten hin geöffnete, Volumenvergrößerungskammer umfasst. Die Volumenvergrößerungsvorrichtung kann fest, zum Beispiel durch Scharniere mit dem Biochip-Träger verbunden sein, sie kann aber auch getrennt von dem Biochip-Träger vorliegen und erst bei Notwendigkeit auf dessen Reaktionskammer aufgesteckt oder sonst wie reversibel befestigt werden. Es ist vorgesehen, dass der mindestens einen Reaktionskammer eines erfindungsgemäßen Biochip-Trägers eine reversibel anbringbare Volumenvergrößerungskammer für diese mindestens eine Reaktionskammer zugeordnet ist, die es gestattet, für bestimmte Arbeitsschritte, beispielsweise Hybridisierungs- oder Waschschritte, die er-findungsgemäße Reaktionskammer mit einem größeren Volumen zu versehen. Die Höhe der Seitenwände der Volumenvergrößerungskammer ist vorzugsweise wesentlich höher, zum Beispiel 5, 10, 15, 20, 25, 30, 40, 50, 60, 70, 80, 90 oder 100 mal höher als die Höhe der Seitenwand der Reaktionskammer bei gleicher Grundfläche der jeweiligen Kammer. Dabei kann selbstverständlich vorgesehen sein, dass wenn der erfindungsgemäße Biochip-Träger mehr als eine Reaktionskammer aufweist, auch für jede einzelne dieser mehreren oder vielen Reaktionskammern, eine derartig reversibel aufsteckbare Volumenvergrößerungskammer vorgesehen ist, wobei diese Mehrzahl der Volumenvergrößerungskammern auch in einer Einheit vorliegen können, beispielsweise in Streifen- oder Matrixform, wobei die Volumenvergrößerungskammern mittels des Rahmenteils miteinander verbunden sind. Die demgemäss in Kombination mit den erfindungsgemäßen Biochip-Trägern vorgesehenen Volumenvergrößerungseinrichtungen zeichnen sich in bevorzugter Ausführungsform dadurch aus, dass sie jeweils ein Rahmenteil und die Volumenvergrößerungskammer bildende Seitenwände aufweisen. Die Seitenwände umschließen seitwärts einen nach oben und unten hin nicht abgeschlossenen, sondern geöffneten Raum oder Volumen. Die mindestens eine Volumenvergrößerungskammer und ein Rahmenteil umfassende Volumenvergrößerungsvorrichtung dient im Wesentlichen dazu, reversibel die an sich eine geringe Höhe aufweisenden Seitenwände der Reaktionskammer in ihrer Höhe zu vergrößern, sodass reversibel die die Reaktionskammer bildenden Seitenwände erhöht und das von ihnen umschlossene Volumen vergrößert wird. In besonders bevorzugter Ausführungsform entspricht der Grundriss des von den Seitenwänden der Volumenvergrößerungskammer umschlossenen Volumens beziehungsweise Fläche dem Grundriss der Fläche des Bodenteils der volumen-zu-vergrößernden Reaktionskammer der Trägerplatte. Ist beispielsweise die Reaktionskammer der Trägerplatte im Grundriss rechteckig oder quadratisch, stehen deren Seitenwände also rechtwinklig zu einander und umschließen demgemäß ein rechteckiges oder quadratisches Bodenteil, so weist auch die Volumenvergrößerungskammer rechtwinklig zueinander angeordnete Seitenwände auf, die eine der Fläche des Bodenteils entsprechende rechteckige oder quadratische Fläche (in Grundriss gesehen) umfassen, wobei das von den Seitenwänden der Volumenvergrößerungskammer umfasste Volumen oder Raum sowohl nach oben als auch nach unten, nämlich zur Reaktionskammer hin, geöffnet ist.

In einer besonders bevorzugten Ausführungsform ist vorgesehen, dass die Volumenvergrößerungseinrichtung auf eine, mehrere oder viele ihr zugeordnete Reaktionskammern gesteckt wird, und zwar derart, dass eine flüssigkeitsdichte Verbindung zwischen den Seitenwänden jeder Volumenvergrößerungskammer und den Seitenwänden jeder einer Volumenvergrößerungskammer zugeordneten Reaktionskammer der Trägerplatte gewährleistet wird. Dies kann beispielsweise geschehen, indem die Volumenvergrößerungsvorrichtung, insbesondere deren Seitenwände aus einem elastischen oder flexiblen Material hergestellt sind, die die vorgenannten Bedingungen gewährleisten. Es kann auch vorgesehen sein, dass die Volumenvergrößerungsvorrichtung beziehungsweise -kammer auf die zugeordnete Reaktionskammer aufgeclipst oder sonst wie verbunden wird, wobei diese Verbindung reversibel sein muss, um das Anbringen und Entfernen zu ermöglichen. Die Volumenvergrößerungsvorrichtung kann beispielsweise aus Silikon, einem Polymer, zum Beispiel Polyurethan, oder sonstigem flüssigkeits-dichtenden Material hergestellt sein.

Die Erfindung betrifft in einer besonders bevorzugten Ausführungsform eine Kombination aus einer wie vorstehend aufgebauten Volumenvergrößerungsvorrichtung, die mindestens eine, zum Beispiel eine Mehrzahl oder Vielzahl von Volumenvergrößerungskammern aufweist, angeordnet in einem Rahmenteil, der beispielsweise als Streifen oder Matrix ausgebildet sein kann, und einem erfindungsgemäßen Biochip-Träger. Der Biochip-Träger kann in bevorzugter Ausführungsform mindestens eine auf einem Sockel angeordnete Reaktionskammer, vorzugsweise mehrere oder eine Vielzahl von auf jeweils einzelnen Sockeln angeordneten Reaktionskammern aufweisen. Diese Ausführungsform ist insofern besonders vorteilhaft, als dass die jeweiligen die Volumenvergrößerungskammer bildenden Seitenwände der Volumenvergrößerungsvorrichtung besonders einfach und besonders flüssigkeitsdicht mit den auf dem Sockel angeordneten Seitenwänden der Reaktionskammer reversibel verbunden werden können.

In einer besonders bevorzugten Ausführungsform ist vorgesehen, dass zwischen Biochip-Träger und der reversibel zuordenbaren Volumenvergrößerungsvorrichtung eine Dichtungsmatte oder Dichtungsmatrix angebracht ist, die eine nochmals verbesserte flüssigkeitsdichte Verbindung zwischen Biochip-Träger und Volumenvergrößerungsvorrichtung gewährleistet. Dies geschieht insbesondere dadurch, dass die vorzugsweise aus einem elastischen flüssigkeitsdichten Material, zum Beispiel einem Polymermaterial hergestellte Dichtungsmatte mit Aussparungen für die mindestens eine Reaktionskammer versehen ist und die Vertiefungen zwischen benachbarten Reaktionskammern eines Biochip-Trägers ausfüllt, so dass bei Aufsetzen der Volumenvergrößerungsvorrichtung auf den Biochip-Träger möglicherweise entstehende Undichtheiten und Kreuzkontamination vermieden werden.

In einer weiteren bevorzugten Ausführungsform ist vorgesehen, dass die Volumenvergrößerungskammern der Volumenvergrößerungsvorrichtung reversibel verschließbar sind, beispielsweise mittels eines den einzelnen Kammern zugeordneten Deckels.

Die Erfindung betrifft selbstverständlich auch einen Kit umfassend einen erfindungsgemäßen Biochip-Träger zusammen mit einer herkömmlichen Mikrotiterplatte.

Die Erfindung betrifft selbstverständlich auch einen Kit umfassend einen erfindungsgemäßen Biochip-Träger zusammen mit einer wie vorstehend beschriebenen Volumenvergrößerungsvorrichtung und einer wie vorstehend beschriebenen Dichtungsmatte.

Die vorliegende Beschreibung betrifft auch die Verwendung des erfindungsgemäßen Biochip-Trägers beziehungsweise des unter Verwendung des erfindungsgemäßen Biochip-Trägers hergestellten Biochips, optional in Kombination mit einer Volumenvergrößerungsvorrichtung, zur Untersuchung eines Analyten in einer Probe und/oder zu dessen Isolierung und/oder Aufreinigung daraus. Im Zusammenhang mit der vorliegenden Erfindung wird unter einem "Analyten" eine Substanz verstanden, bei der Art und Menge ihrer Einzelbestandteile bestimmt und/oder die aus Gemischen abgetrennt werden soll. Insbesondere handelt es sich bei dem Analyten um Proteine, Nucleinsäuren, Kohlenhydrate und ähnliche Verbindungen. In bevorzugter Ausführungsform der Erfindung ist der Analyt ein Protein, Peptid, Antigen oder eine Nucleinsäure. Unter einer "Probe" wird eine wässrige oder organische Lösung, Emulsion, Dispersion oder Suspension verstanden, die einen vorstehend definierten Analyten in isolierter und aufgereinigter Form oder als Bestandteil eines komplexen Gemisches unterschiedliche Substanzen enthält. Bei einer Probe kann es sich insbesondere um eine biologische Flüssigkeit, wie Blut, Lymphe, Gewebeflüssigkeit etc. handeln, also eine Flüssigkeit, die einem lebenden oder toten Organismus, Organ oder Gewebe entnommen wurde. Eine Probe kann bereits Aufreinigungsschritten unterworfen worden sein, kann aber auch ungereinigt vorliegen.

Die vorliegende Beschreibung betrifft daher auch die Verwendung des erfindungsgemäßen Biochip-Trägers beziehungsweise eines unter Verwendung des erfindungsgemäßen Biochip-Trägers hergestellten Biochips, optional in Kombination mit einer Volumenvergrößerungsvorrichtung, zur Durchführung von Analyse- und/oder Detektionsverfahren, wobei es sich bei diesen Verfahren beispielsweise um Massenspektroskopie, Fluoreszenz- oder UV-VIS-Spektroskopie, Fluoreszenz- oder Lichtmikroskopie, Wellenleiterspektroskopie oder ein elektrisches Verfahren wie Impedanzspektroskopie handelt.

Die vorliegende Beschreibung betrifft ebenfalls die Verwendung des erfindungsgemäßen Biochip-Trägers beziehungsweise des unter Verwendung des erfindungsgemäßen Biochip-Trägers hergestellten Biochips, optional in Kombination mit einer Volumenvergrößerungsvorrichtung, zum Nachweis und/oder zur Isolierung biologischer Moleküle. Beispielsweise kann ein Biochip-Träger beziehungsweise Biochip, optional in Kombination mit einer Volumenvergrößerungsvorrichtung, der eine vorzugsweise einzelsträngige Nucleinsäure in immobilisierter Form aufweist, zum Nachweis einer komplementären Nucleinsäure in einer Probe und/oder zur Isolierung dieser komplementären Nucleinsäure eingesetzt werden. Ein erfindungsgemäßer Biochip-Träger oder Biochip, optional in Kombination mit einer Volumenvergrößerungsvorrichtung, der ein Protein in immobilisierter Form aufweist, kann beispielsweise zum Nachweis und/oder zur Isolierung eines mit dem immobilisierten Protein in Wechselwirkung tretenden zweiten Proteins aus einer Probe eingesetzt werden.

Die vorliegende Beschreibung betrifft auch die Verwendung des erfindungsgemäßen Biochip-Trägers beziehungsweise des unter Verwendung des Biochip-Trägers hergestellten Biochips, optional in Kombination mit einer Volumenvergrößerungsvorrichtung, zur Entwicklung von pharmazeutischen Präparaten. Sie betrifft ebenfalls die Verwendung des erfindungsgemäßen Biochip-Trägers beziehungsweise des unter Verwendung des erfindungsgemäßen Biochip-Trägers hergestellten Biochips, optional in Kombination mit einer Volumenvergrößerungsvorrichtung, zur Untersuchung der Wirkungen und/oder Nebenwirkungen von pharmazeutischen Präparaten. Die erfindungsgemäßen Biochip-Träger beziehungsweise die unter Verwendung des erfindungsgemäßen Biochip-Trägers hergestellten Biochips, optional in Kombination mit einer Volumenvergrößerungsvorrichtung, lassen sich ebenfalls zur Diagnose von Krankheiten, beispielsweise zur Identifizierung von Krankheitserregern und/oder zur Identifizierung von mutierten Genen, die zur Entstehung von Krankheiten führen, verwenden. Eine weitere Verwendungsmöglichkeit des erfindungsgemäßen Biochip-Trägers beziehungsweise Biochips, optional in Kombination mit einer Volumenvergrößerungsvorrichtung, besteht bei der Untersuchung der mikrobiologischen Kontamination von beispielsweise Nahrungsmitteln, Trinkwasser, Abwasser oder Fermentern.

Weitere vorteilhafte Ausgestaltungen der Erfindung ergeben sich aus den Unteransprüchen.

Der erfindungsgemäße Biochip-Träger wird anhand von Ausführungsbeispielen und der dazugehörigen Figuren näher erläutert.

Es zeigen:
- Figur 1: eine schematische Darstellung eines erfindungsgemäßen Biochip-Trägers in Draufsicht gesehen,
- Figur 2: eine schematische Darstellung zweier alternativer Ausführungsformen des erfindungsgemäßen Biochip-Trägers im Querschnitt,
- Figur 3: eine Draufsicht auf sieben alternative Ausführungsformen des erfindungsgemäßen Biochip-Trägers,
- Figur 4: eine seitliche Ansicht zweier alternativer Ausführungsformen des erfindungsgemäßen Biochip-Trägers und einer Mikrotiterplatte mit eingesetzten erfindungsgemäßen Biochip-Trägern,
- Figur 5: eine seitliche Ansicht einer weiteren alternativen Ausführungsform des erfindungsgemäßen Biochip-Trägers,
- Figur 6: zwei seitliche Ansichten weiterer bevorzugter Ausführungsformen des erfindungsgemäßen Biochip-Trägers, wobei die Reaktionskammern auf Sockeln angeordnet sind,
- Figur 7: eine Ausführungsform einer erfindungsgemäßen Volumenvergrößerungsvorrichtung in Streifenform,
- Figur 8: eine seitliche Ansicht einer auf einem erfindungsgemäßen Biochip-Träger im SBS-Format aufsteckbaren Volumenvergrößerungseinrichtung, wobei der Biochip-Träger auf einer herkömmlichen Mikrotiterplatte aufsteckbar ist, und
- Figur 9: einen Biochip-Träger im SBS-Format und eine diesem zugeordnete Volumenvergrößerungsvorrichtung im SBS-Format, wobei zwischen diesen eine Dichtungsmatte im SBS-Format angeordnet ist.

Im folgenden weisen bau- beziehungsweise funktionsgleiche Teile, Elemente oder Vorrichtungen gleiche Bezugsziffern auf.

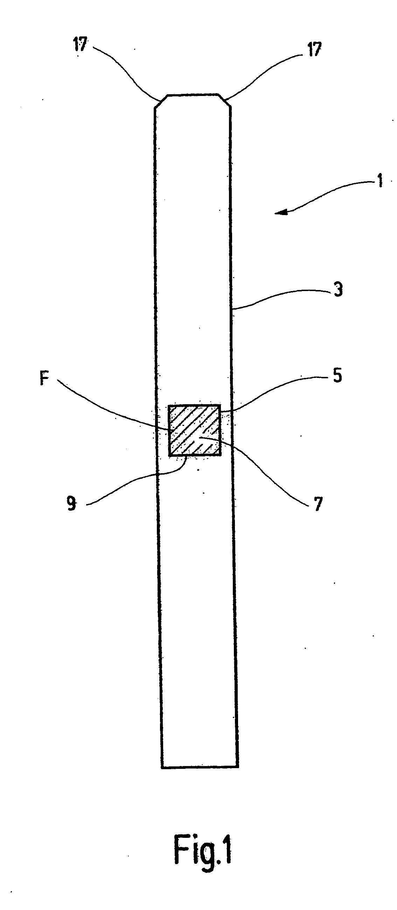

Die Figur 1 zeigt in schematischer Form einen Biochip-Träger 1 mit einer rechteckigen, teilweise abgeschrägte Ecken 17 aufweisenden Trägerplatte 3. Auf der Trägerplatte 3 ist eine Reaktionskammer 5 angeordnet, die durch das Bodenteil 7 mit der schraffiert dargestellten Fläche F und das Bodenteil 7 seitwärts begrenzende beziehungsweise umfassende Seitenwände 9 gebildet wird. Die im Grundriss quadratische Reaktionskammer 5 ist nach oben hin offen. Die Trägerplatte 3 weist zwei abgeschrägte Ecken 17 auf, die eine eindeutige Orientierung des Biochip-Trägers 1, beispielsweise beim Einsetzen in eine Mikrotiterplatte, erlauben.

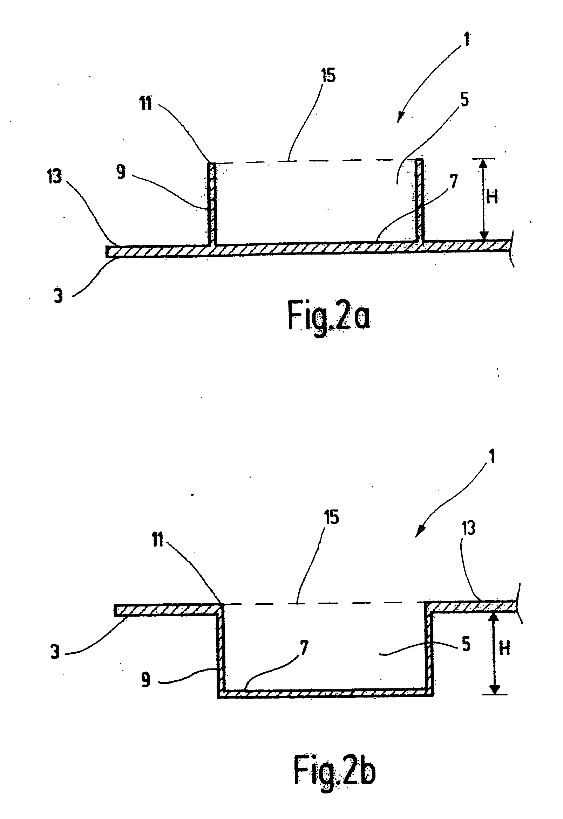

Die Figur 2 zeigt in schematischer Form einen Querschnitt durch zwei alternative Ausführungsformen des erfindungsgemäßen Biochip-Trägers 1. Die Figur 2a zeigt eine Ausführungsform des erfindungsgemäßen Biochip-Trägers 1, bei der das Bodenteil 7 der Reaktionskammer 5 durch die Trägerplatte 3 gebildet wird. Die Seitenwände 9 der Reaktionskammer 5 mit einer Höhe H sind als Erhebungen auf der Trägerplatte 3 ausgebildet. Die nach oben hin offene Reaktionskammer 5 weist die Öffnung 15 auf. Bei dieser Ausführungsform liegt die Oberkante 11 der Reaktionskammer 5 in einer Ebene oberhalb der Oberkante 13 der Trägerplatte 3. Die Figur 2b zeigt eine Ausführungsform des erfindungsgemäßen Biochip-Trägers 1, bei der die Oberkante 11 der Reaktionskammer 3 und die Oberkante 13 der Trägerplatte 3 in der gleichen Ebene liegen. In dieser Ausführungsform ist die Reaktionskammer 5 innerhalb und/oder unterhalb der Trägerplatte 3 als Vertiefung angeordnet, wobei die Öffnung 15 der Reaktionskammer 5 mit der Oberkante 13 der Trägerplatte 3 abschließt.

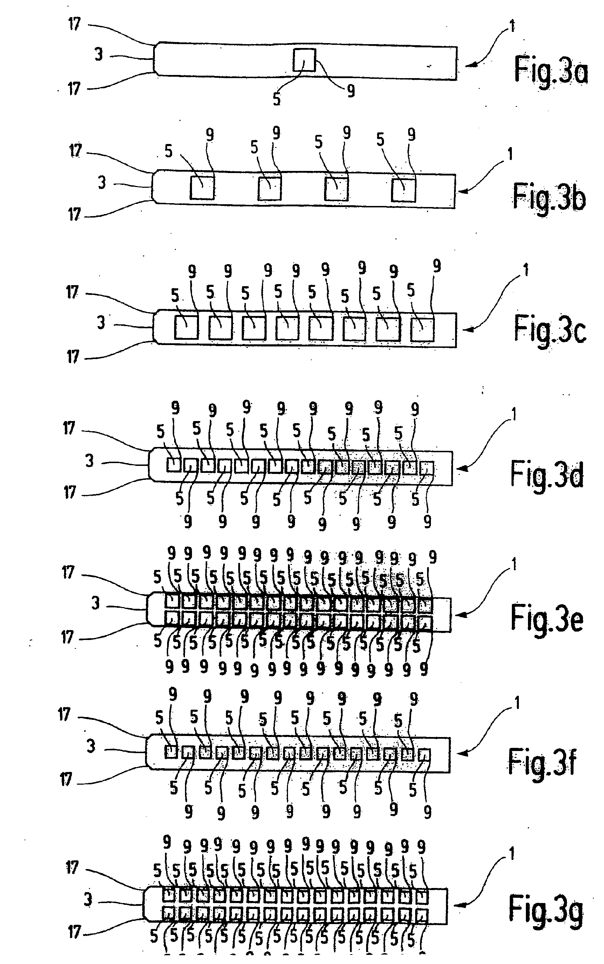

Die Figur 3 zeigt alternative Ausführungsformen des erfindungsgemäßen Biochip-Trägers 1 in Draufsicht gesehen. Der erfindungsgemäße Biochip-Träger 1 ist jeweils in Streifenform ausgeführt, wobei die Länge des Biochip-Trägers 1 dessen, vorzugsweise passgenaues, Einsetzen in eine Mikrotiterplatte nach SBS-Standard erlaubt. Die Breite des Biochip-Trägers 1 beträgt jeweils 9 mm. Die auf der Trägerplatte 3 angeordneten Reaktionskammern 5 sind jeweils nach oben hin offen. Die jeweilige Trägerplatte 3 der gezeigten Ausführungsformen weist jeweils zwei abgeschrägte Ecken 17 auf, die eine eindeutige Orientierung des Biochip-Trägers 1, beispielsweise beim Einsetzen in eine Mikrotiterplatte, erlauben.

Figur 3a zeigt eine Ausführungsform, bei der eine Reaktionskammer 5 auf der Trägerplatte 3 des Biochip-Trägers 1 angeordnet ist. Die Reaktionskammer 5 weist einen quadratischen Grundriss mit einer Seitenlänge von 6 mm auf. Bei einer Höhe der Seitenwände 9 der Reaktionskammer 5 von 0,6 mm beträgt das Verhältnis des Zahlenwertes der Bodenteil-Fläche zum Zahlenwert der Höhe der Seitenwände (36(mm²):0,6(mm)=60) 60. Bei einer Höhe der Seitenwände 9 der Reaktionskammer 5 von 0,5 mm beträgt das Verhältnis des Zahlenwertes der Bodenteil-Fläche zum Zahlenwert der Höhe der Seitenwände 72. Bei einer Höhe der Seitenwände 9 der Reaktionskammer 5 von 0,4 mm beträgt das Verhältnis des Zahlenwertes der Bodenteil-Fläche zum Zahlenwert der Höhe der Seitenwände 90.

Figur 3b zeigt eine Ausführungsform, bei der 4 gleichmäßig beabstandete Reaktionskammern 5 in einer Reihe auf der Trägerplatte 3 des Biochip-Trägers 1 angeordnet sind. Die 4 Reaktionskammern 5 weisen jeweils einen quadratischen Grundriss mit einer Seitenlänge von jeweils 6 mm auf.

Figur 3c zeigt eine Ausführungsform, bei der 8 gleichmäßig beabstandete Reaktionskammern 5 in einer Reihe auf der Trägerplatte 3 des Biochip-Trägers 1 angeordnet sind. Die 8 Reaktionskammern 5 weisen jeweils einen quadratischen Grundriss mit einer Seitenlänge von jeweils 6 mm auf.

Figur 3d zeigt eine Ausführungsform, bei der 16 gleichmäßig beabstandete Reaktionskammern 5 in einer Reihe auf der Trägerplatte 3 des Biochip-Trägers 1 angeordnet sind. Die 16 Reaktionskammern 5 weisen jeweils einen quadratischen Grundriss mit einer Seitenlänge von jeweils 3,5 mm auf. Bei einer Höhe der Seitenwände 9 der Reaktionskammer 5 von 0,4 mm beträgt das Verhältnis des Zahlenwertes der Bodenteil-Fläche zum Zahlenwert der Höhe der Seitenwände etwa 30. Bei einer Höhe der Seitenwände 9 der Reaktionskammer 5 von 0,3 mm beträgt das Verhältnis des Zahlenwertes der Bodenteil-Fläche zum Zahlenwert der Höhe der Seitenwände etwa 40. Bei einer Höhe der Seitenwände 9 der Reaktionskammer 5 von 0,2 mm beträgt das Verhältnis des Zahlenwertes der Bodenteil-Fläche zum Zahlenwert der Höhe der Seitenwände etwa 61.

Figur 3e zeigt eine Ausführungsform, bei der 32 gleichmäßig beabstandete Reaktionskammern 5 in zwei parallelen Reihen auf der Trägerplatte 3 des Biochip-Trägers 1 angeordnet sind. Die 32 Reaktionskammern 5 weisen jeweils einen quadratischen Grundriss mit einer Seitenlänge von jeweils 3,5 mm auf.

Figur 3f zeigt eine Ausführungsform, bei der 16 gleichmäßig beabstandete Reaktionskammern 5 in einer Reihe auf der Trägerplatte 3 des Biochip-Trägers 1 angeordnet sind. Die 16 Reaktionskammern 5 weisen jeweils einen quadratischen Grundriss mit einer Seitenlänge von jeweils 3 mm auf. Bei einer Höhe der Seitenwände 9 der Reaktionskammer 5 von 0,3 mm beträgt das Verhältnis des Zahlenwertes der Bodenteil-Fläche zum Zahlenwert der Höhe der Seitenwände 30. Bei einer Höhe der Seitenwände 9 der Reaktionskammer 5 von 0,2 mm beträgt das Verhältnis des Zahlenwertes der Bodenteil-Fläche zum Zahlenwert der Höhe der Seitenwände 45.

Figur 3g zeigt eine Ausführungsform, bei der 32 gleichmäßig beabstandete Reaktionskammern 5 in zwei parallelen Reihen auf der Trägerplatte 3 des Biochip-Trägers 1 angeordnet sind. Die 32 Reaktionskammern 5 weisen jeweils einen quadratischen Grundriss mit einer Seitenlänge von jeweils 3 mm auf.

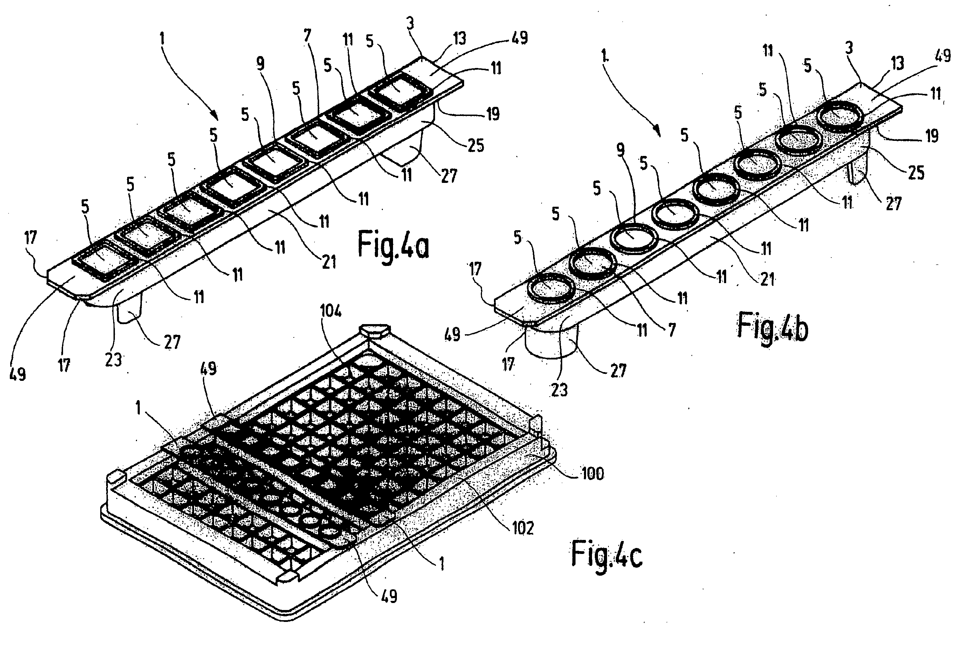

Die Figur 4 zeigt eine seitliche Ansicht zweier alternativer Ausführungsformen des erfindungsgemäßen Biochip-Trägers 1, in denen das Bodenteil 7 in einer Ebene mit der Oberkante 17 der Trägerfläche 3 liegt und einer Mikrotiterplatte mit eingesetzten erfindungsgemäßen Biochip-Trägern 1.

Figur 4a zeigt einen in Streifenform ausgeführten Biochip-Träger 1, bei dem acht gleichmäßig beabstandete Reaktionskammern 5 in einer Reihe auf der Trägerplatte 3 angeordnet sind. Bei dieser Ausführungsform liegt die Oberkante 11 der durch die als Erhebungen ausgeführten Seitenwände 9 gebildeten Reaktionskammern 5 in einer Ebene oberhalb der Oberkante 13 der Trägerplatte 3. Die nach oben hin offenen Reaktionskammern 5 weisen jeweils einen quadratischen Grundriss auf. Die Trägerplatte 3 weist zwei abgeschrägte Ecken 17 auf, die eine Orientierung des Biochip-Trägers 1 ermöglichen. An der Unterkante 19 der Trägerplatte 3 ist ein Rahmenelement 21 ausgebildet, das aus einer umlaufenden hohlen Wand gebildet wird. Die nicht gezeigte Innenwand des Rahmenelements 21 kann durch ebenfalls nicht gezeigte Stege verbunden sein. An den Enden 23 und 25 des Rahmenelements 21 sind jeweils hervorspringende Fortsätze 27 angeordnet, die beim Einsetzen des Biochip-Trägers 1 in eine Mikrotiterplatte in entsprechende Aussparungen der Mikrotiterplatte einrasten und so zusammen mit den auf dem Rahmen einer Mikrotiterplatte aufliegenden Auflagefläche 49 den Biochip-Träger 1 in, beziehungsweise auf der Mikrotiterplatte fixieren. Dargestellt sind zwei endständige Auflageflächen 49 der Trägerplatte 3, die eine Auflage der Trägerfläche auf zum Beispiel einer herkömmlichen Mikrotiterplatte erlauben.

Figur 4b zeigt einen in Streifenform ausgeführten Biochip-Träger 1, bei dem ebenfalls acht gleichmäßig beabstandete Reaktionskammern 5 in einer Reihe auf der Trägerplatte 3 angeordnet sind, wobei die Reaktionskammer 5 durch als Erhebungen ausgeführte Seitenwände 9 und ein in der Ebene der Oberfläche der Trägerplatte 3 liegendes Bodenteil 7 gebildet wird. Auch bei dieser Ausführungsform liegt die Oberkante 11 der Reaktionskammern 5 in einer Ebene oberhalb der Oberkante 13 der Trägerplatte 3. Die nach oben hin offenen Reaktionskammern 5 weisen in dieser Ausführungsform jeweils einen kreisförmigen Grundriss auf. Auch in dieser Ausführungsform ist an der Unterkante 19 der Trägerplatte 3 ein aus einer umlaufenden hohlen Wand gebildetes Rahmenelement 21 angebracht. An den Enden 23 und 25 des Rahmenelements 21 sind jeweils hervorspringende Fortsätze 27 angeordnet, die beim Einsetzen des Biochip-Trägers 1 in eine Mikrotiterplatte in entsprechende Aussparungen der Mikrotiterplatte einrasten und so zusammen mit den auf dem Rahmen einer Mikrotiterplatte aufliegenden Auflageflächen 49 den Bio-Chip-Träger 1 in und auf der Mikrotiterplatte fixieren.

Figur 4c zeigt eine herkömmliche Mikrotiterplatte 100, in die die in den Figuren 4a und 4b dargestellten Biochip-Träger 1 eingesetzt sind, und zwar mit ihrer Länge senkrecht zur Länge der Mikrotiterplatte 100. Der Mikrochip-Träger 1 deckt die Kavitäten 104 der Mikrotiterplatte 100 nach oben hin ab und ist mit den Auflageflächen 49 auf dem Rahmen 102 der Mikrotiterplatte 100 fixiert.

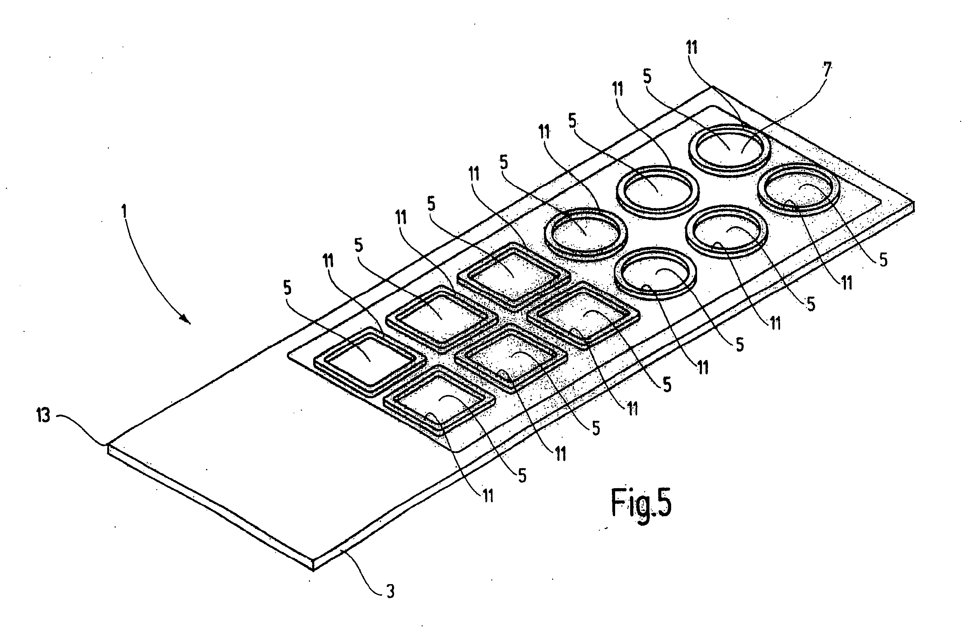

Figur 5 zeigt eine weitere Ausführungsform des erfindungsgemäßen Biochip-Trägers 1. Der dargestellte Biochip-Träger 1 ist in Form eines Standard-Objektträgers ausgebildet. Auf der Trägerplatte 3 sind 12 gleichmäßig beabstandete, nach oben hin offene Reaktionskammern 5 in zwei parallelen Reihen angeordnet. Auch bei dieser Ausführungsform liegt die Oberkante 11 der durch die als Erhebungen ausgebildeten Seitenwände 9 seitwärts begrenzten Reaktionskammern 5 in einer Ebene oberhalb der Oberkante 13 der Trägerplatte 3. Das Bodenteil 7 liegt in einer Ebene mit der Oberkante 13 der Trägerplatte 3. Jeweils drei der in einer Reihe angeordneten Reaktionskammern 5 weisen einen kreisförmigen Grundriss auf, während die drei anderen Reaktionskammern 5 einer Reihe einen quadratischen Grundriss haben.

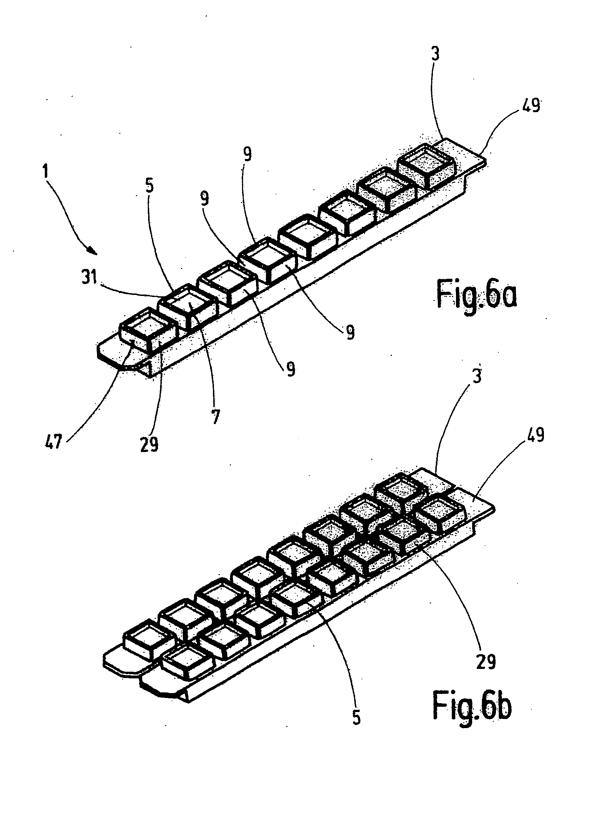

Figur 6a zeigt in seitlicher Ansicht einen erfindungsgemäßen Biochip-Träger 1, der in Streifenform ausgeführt ist und 8 in Reihen angeordnete Reaktionskammern 5 aufweist, wobei die Reaktionskammern 5 jeweils im Grundriss quadratisch ausgebaut sind und vier rechtwinklig zueinander angeordnete Seitenwände 9, welche als Erhebungen ausgeführt sind, aufweist. Diese Seitenwände 9 umfassen zusammen mit dem planaren, im Grundriss gesehen quadratischen Bodenteil 7 die nach oben hin offene Reaktionskammer 5. Die Reaktionskammer 5 ist auf einem Sockel 29 angeordnet, wobei die obere Fläche 31 des Sockels 29 gleichzeitig das Bodenteil 7 der Reaktionskammer 5 darstellt. Im Grundriss gesehen weist der Sockel 29 die gleiche Geometrie und Abmessungen wie das Bodenteil 7 der Reaktionskammer 5 auf. Die Seitenwände 9 der Reaktionskammer 5 stellen gleichsam eine Verlängerung oder Fortsetzung der Seitenwände 47 des Sockels 29 in ein und derselben Ebene dar. Sowohl die Reaktionskammer 5 als auch der Sockel 29 sind einstückig und als integraler Bestandteil der Trägerplatte 3 ausgebildet.

Die Figur 6b stellt zwei miteinander verbundene Biochip-Träger 1, gemäß Figur 6a, dar. Die als Streifen ausgeführten Biochip-Träger 1 der Figur 6a können gemäß dieser Figur reversibel oder fest miteinander verbunden sein, und so in Form einer 2 x 8 Reaktionskammermatrix in eine Mikrotiterplatte eingesetzt werden.

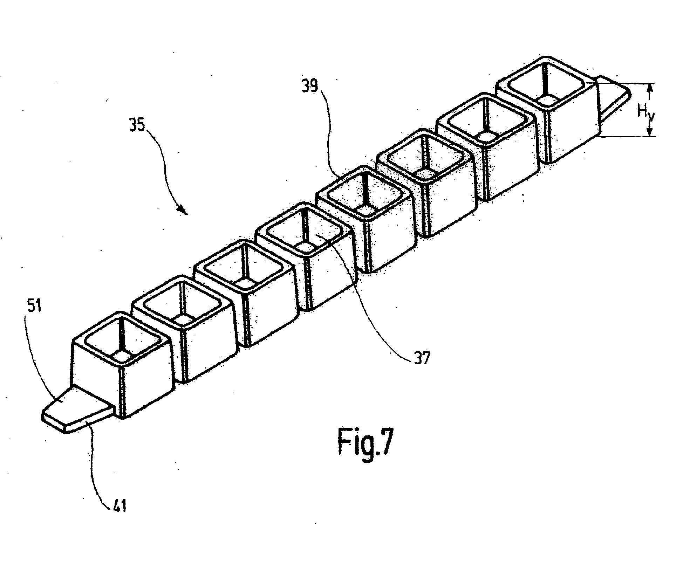

Figur 7 stellt eine Volumenvergrößerungsvorrichtung 35 mit in Streifenform in Reihe hintereinander angeordneten, im Grundriss gesehen, quadratischen Volumenvergrößerungskammern 37, dar. Die Volumenvergrößerungskammern sind nach oben und nach unten (nicht dargestellt) hin geöffnet, sodass die Kammer 37 durch die rechtwinklig zueinander angeordneten jeweils vier Seitenwände 39 gebildet werden. Die Höhe Hᵥ der Seitenwände 39 ist erheblich größer als die Höhe H der Seitenwände 9 der Reaktionskammer 5 eines erfindungsgemäßen Biochip-Trägers. Das die einzelnen Volumenvergrößerungskammern 37 verbindende Rahmenteil 41 ist an den beiden Enden des Streifens als Fortsatz 51 ausgebildet, welche das Positionieren und Fixieren der Volumenvergrößerungsvorrichtung 35 auf einem Biochip-Träger 1 der Erfindung ermöglicht.

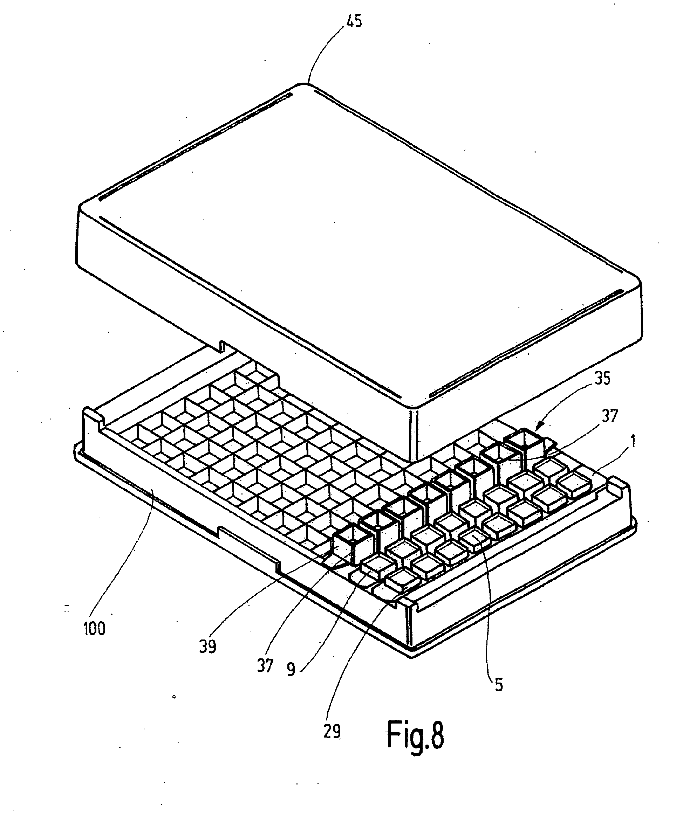

Figur 8 zeigt eine herkömmliche Mikrotiterplatte 100 sowie einen auf diesen aufbringbaren Biochip-Träger 1. Der Biochip-Träger 1 ist ein Biochip-Träger, der auf Sockeln 29 angeordnete Reaktionskammer 5, wie beispielsweise in Figur 6a und 6b gezeigt, aufweist. Dargestellt ist ferner eine Volumenvergrößerungseinrichtung 35 gemäß Figur 7, die reversibel auf den Biochip-Träger 1 aufgesteckt werden kann. Dabei wird jeder Reaktionskammer 5 des Biochip-Trägers 1 eine Volumenvergrößerungskammer 37 der Volumenvergrößerungsvorrichtung 35 so zugeordnet, dass sich das Volumen der Reaktionskammer 5 im Wesentlichen um das Volumen der Volumenvergrößerungskammer 37 reversibel erhöht. Dies wird dadurch erreicht, dass die Volumenvergrößerungsvorrichtung 35 so auf den Biochip-Träger 1 gesteckt wird, dass die Seitenwände 39 der Volumenvergrößerungskammer 37 flüssigkeitsdicht auf die Seitenwände 9 des Biochip-Träger 1 gesteckt werden. Dargestellt ist ferner ein Deckel 45, der reversibel aufsteckbar die Reaktionskammer 5 beziehungsweise die Volumenvergrößerungskammer 37 nach oben hin verschließen kann.

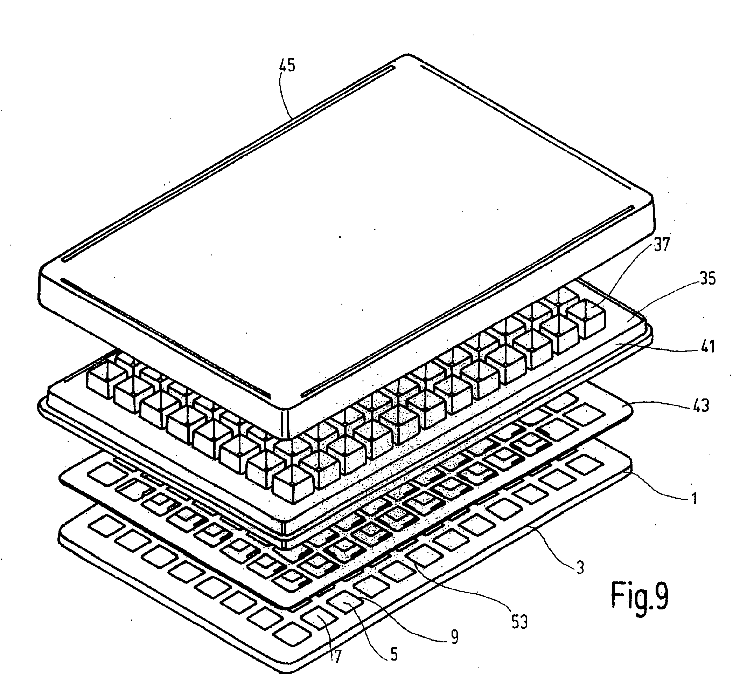

Figur 9 stellt einen erfindungsgemäßen Biochip-Träger 1 in Matrixform und im SBS-Standard dar. Dargestellt sind die Reaktionskammern 5, die aus einem Bodenteil 7 und als Erhebungen ausgeführten Seitenwänden 9 gebildet werden. Dargestellt ist ferner eine Volumenvergrößerungsvorrichtung 35 umfassend den 96 Reaktionskammern 5 des Biochip-Trägers 1 entsprechende 96 Volumenvergrößerungskammern 37. Die Abmessungen der Volumenvergrößerungsvorrichtung 35 umfassend die 96 Volumenvergrößerungskammern 37 in einem Rahmenteil 41 entsprechen denen einer Mikrotiterplatte im SBS-Format. Diese Volumenvergrößerungsvorrichtung 35 ist reversibel auf die Trägerplatte 3 des Biochip-Trägers 1 aufbringbar beziehungsweise aufsteckbar und erhöht so, da die einzelnen Volumenvergrößerungskammern 37 sowohl nach oben als auch nach unten vollständig geöffnet sind, reversibel das jeweilige Volumen der Reaktionskammern 5 des Biochip-Trägers 1. Zur Erhöhung der Flüssigkeitsdichtung zwischen Reaktionskammer 5 des Biochip-Trägers 1 und der Volumenvergrößerungskammer 37 stellt die Figur 9 eine zwischen diese beiden Vorrichtungen anordnenbare Dichtungsmatrix oder Dichtungsmatte 43 dar. Diese, vorzugsweise aus einem elastischen und flüssigkeitsdichten, zum Beispiel polymeren Material hergestellte Dichtungsmatte 43 ist so ausgeformt, dass sie auf den Biochip-Träger 1 aufgelegt werden kann, dabei die Grundrisse der Reaktionskammern 5 ausspart und die Zwischenräume 53 zwischen den Reaktionskammern 5 belegt. Die Dichtungsmatte 43 passt sich demgemäss genau in die Vertiefungen 53 zwischen den Reaktionskammern 5 ein und erhöht die Dichtigkeit zwischen der Volumenvergrößerungsvorrichtung 35 und dem Biochip-Träger 1. Dargestellt ist ferner ein Deckel 45, der reversibel aufsteckbar die Reaktionskammer 5 beziehungsweise die Volumenvergrößerungskammer 37 nach oben hin verschließen kann. Allgemein gilt: Der Deckel 45 kann auch nur einzelnen oder mehreren der Reaktionskammern zugeordnet sein, muss also nicht alle Kammern des Biochip-Trägers abdecken.

## Patentansprüche

1. Biochip-Träger umfassend eine Trägerplatte (3) mit mindestens einer dreidimensionalen Reaktionskammer (5), wobei die Reaktionskammer (5) von einem Bodenteil (7) und das nach oben hin geöffnete Volumen der Reaktionskammer (5) seitwärts umfassenden Seitenwänden (9) gebildet ist, wobei das Verhältnis zwischen der Fläche (F) des Bodenteils (7) und der Höhe (H) der Seitenwände (9) größer oder gleich 30, vorzugsweise größer als 50 ist, und wobei das Bodenteil (7) und/oder mindestens eine Seitenwand (9) der mindestens einen Reaktionskammer (5) als Bindematrix mit einer funktionellen Gruppe, die die Bindung eines natürlichen oder synthetischen Moleküls, insbesondere eines biologisch aktiven Moleküls ermöglicht, ausgeführt ist, und wobei der mindestens einen Reaktionskammer (5) eine Volumenvergrößerungskammer (37) reversibel zugeordnet ist, wobei die Volumenvergrößerungskammer (37) so auf den Biochip-Träger (1) aufbringbar ist, dass eine flüssigkeitsdichte Verbindung zwischen den Seitenwänden (9) der Reaktionskammer (5) und den Seitenwänden (39) der Volumenvergrößerungskammer (37) gebildet wird.

2. Biochip-Träger nach Anspruch 1, wobei die Oberkante (11) der mindestens einen Reaktionskammer (5) den höchsten Teil des Biochip-Trägers (1) darstellt.

3. Biochip-Träger nach Anspruch 1 oder 2, wobei die Oberkante (11) der mindestens einen Reaktionskammer (5) in einer Ebene oberhalb der Oberkante (13) der Trägerplatte (3) liegt.

4. Biochip-Träger nach Anspruch 3, wobei die Trägerplatte (3) das Bodenteil (7) der mindestens einen Reaktionskammer (5) ausbildet und die Seitenwände (9) der Reaktionskammer (5) als Erhebungen auf der Trägerplatte (3) ausgebildet sind.

5. Biochip-Träger nach einem der Ansprüche 1 bis 4, wobei die mindestens eine Reaktionskammer (5) auf einem Sockel (29) angeordnet ist.

6. Biochip-Träger nach einem der Ansprüche 1 bis 5, wobei der Sockel (29) im Grundriss die Form des Grundrisses der Reaktionskammer (5) aufweist.

7. Biochip-Träger nach einem der Ansprüche 1 bis 6, wobei die obere Fläche (31) des Sockels (29) das Bodenteil (7) der Reaktionskammer (5) darstellt.

8. Biochip-Träger nach einem der Ansprüche 1 bis 7, wobei die Seitenwände (33) des Sockels (29) in einer Ebene mit den Seitenwänden (9) der Reaktionskammer (5) liegen.

9. Biochip-Träger nach einem der Ansprüche 1 bis 8, wobei die Seitenwände (9) der Reaktionskammer (5) als Erhebungen auf dem Sockel (29) ausgebildet sind.

10. Biochip-Träger nach Anspruch 1 oder 2, wobei die Oberkante (11) der mindestens einen Reaktionskammer (5) und die Oberkante (13) der Trägerplatte (3) in der gleichen Ebene liegen.

11. Biochip-Träger nach Anspruch 10, wobei die mindestens eine Reaktionskammer (5) innerhalb und/oder unterhalb der Trägerplatte (3) als Vertiefung angeordnet ist.

12. Biochip-Träger nach einem der Ansprüche 1 bis 11, wobei das Bodenteil (7) der mindestens einen Reaktionskammer (5) in Draufsicht gesehen die Form eines Kreises, Rechtecks, Quadrates, Sechsecks, Polygons oder einer Ellipse aufweist.

13. Biochip-Träger nach einem der Ansprüche 1 bis 12, wobei die Trägerplatte (3) Abmessungen aufweist, die das Einsetzen des Biochip-Trägers (1) in eine Mikrotiterplatte mit Abmessungen nach SBS (Society of Biomolecular Screening)-Standard erlauben.

14. Biochip-Träger nach Anspruch 13, wobei der Biochip-Träger (1) 1, 2, 4, 8, 12 oder jeweils ganzzahlige Vielfache von 8 oder 12 Reaktionskammern (5) aufweist.

15. Biochip-Träger nach einem der Ansprüche 1 bis 13, wobei die Trägerplatte (3) als Streifen ausgeführt ist.

16. Biochip-Träger nach Anspruch 15, wobei die Trägerplatte (3) die Abmessungen eines Standard-Objektträgers aufweist.

17. Biochip-Träger nach einem der vorhergehenden Ansprüche, wobei die Trägerplatte (3) als Streifen mit 4 oder jeweils ganzzahligen Vielfachen davon hintereinander angeordneten beabstandeten Reaktionskammern (5) ausgeführt ist.

18. Biochip-Träger nach einem der vorhergehenden Ansprüche, wobei die mindestens eine Reaktionskammer (5) ein kreisförmiges Bodenteil (7) mit einem Durchmesser von 6 mm und eine Seitenwand-Höhe (H) von 0,5 mm aufweist.

19. Biochip-Träger nach einem der vorhergehenden Ansprüche, wobei die mindestens eine Reaktionskammer (5) ein quadratisches Bodenteil mit einer Seitenlänge von 6 mm und eine Seitenwand-Höhe (H) von 0,5 mm aufweist.

20. Biochip-Träger nach einem der Ansprüche 1 bis 19, wobei die Trägerplatte (3) und die mindestens eine Reaktionskammer (5) aus Kunststoff bestehen.

21. Biochip-Träger nach Anspruch 20, wobei es sich bei dem Kunststoff um Styrol-Maleinsäureanhydrid-Copolymer (SMA), Cycloolefin-Copolymer (COC), Cycloolefinpolymer (COP), Acrylbutadienstyrol, Polyamid, Polycarbonat, Polyester, Polymethylmethacrylat, Polypropylen, Polystyrol oder Styrolacrylnitril handelt.

22. Biochip-Träger nach einem der Ansprüche 1 bis 21, wobei das Bodenteil (7) der mindestens einen Reaktionskammer (5) aus einem Polymer, Glas, Membran oder Hybrid davon besteht, das an der Oberfläche mindestens eine funktionelle Gruppe aufweist, die ein biologisch aktives Molekül binden kann.

23. Biochip-Träger nach Anspruch 22, wobei die funktionelle Gruppe unter Verwendung eines chemischen Funktionalisierungsverfahrens auf die Polymer-Oberfläche aufgebracht ist.

24. Biochip-Träger nach Anspruch 23, wobei das Polymer des Bodenteils ein Polymer mit aromatischen Resten ist, an dessen Oberfläche Chloromethyl-Gruppen angelagert sind.

25. Biochip-Träger nach Anspruch 24, wobei das Polymer Polystyrol ist.

26. Biochip-Träger nach Anspruch 24 oder 25, wobei die Chloromethyl-Gruppe des Polymers durch N- oder O-Nucleophile substituiert ist.

27. Biochip-Träger nach Anspruch 26, wobei die Chloromethyl-Gruppe durch eine Aldehyd-Gruppe substituiert ist.

28. Biochip-Träger nach Anspruch 26, wobei die Chloromethyl-Gruppe durch eine Carbonsäure substituiert ist.

29. Biochip-Träger nach Anspruch 23, wobei das Polymer des Bodenteils ein gesättigtes Polymer ist, dessen Oberfläche chloriert ist.

30. Biochip-Träger nach Anspruch 29, wobei das chlorierte Polymer ein chloriertes Cycloolefin-Copolymer, insbesondere chloriertes TOPAS ist.

31. Biochip-Träger nach Anspruch 22, wobei die funktionelle Gruppe unter Verwendung eines Plasma-Funktionalisierungsverfahrens auf die Polymer-Oberfläche aufgebracht ist.

32. Biochip-Träger nach Anspruch 31, wobei die Oberfläche Carbonsäure-, Aldehyd/Keton-, Amin-, Epoxy- und/oder Halogen-Funktionen aufweist.

33. Biochip-Träger nach Anspruch 22, wobei die funktionelle Gruppe unter Verwendung einer photoinduzierten Oberflächenfunktionalisierung auf die Polymer-Oberfläche aufgebracht ist.

34. Biochip-Träger nach Anspruch 33, wobei die funktionelle Gruppe durch Umsetzung von Anthrachinon mit einem Donor und anschließende photochemische Fixierung auf die Polymer-Oberfläche aufgebracht ist.

35. Biochip-Träger nach Anspruch 33, wobei die funktionelle Gruppe durch photochemisches Graften auf die Polymer-Oberfläche aufgebracht ist.

36. Biochip-Träger nach Anspruch 22, wobei die funktionelle Gruppe unter Verwendung von Propfpolymerisation auf die Polymer-Oberfläche aufgebracht ist.

37. Biochip-Träger nach einem der Ansprüche 22 bis 36, wobei am Bodenteil (7) der Reaktionskammer (5) ein biologisch aktives Molekül gebunden ist.

38. Biochip-Träger nach Anspruch 37, wobei das biologisch aktive Molekül ein Protein, eine Nucleinsäure oder ein PNA-Molekül ist.

39. Biochip-Träger nach einem der vorhergehenden Ansprüche, wobei jeder Reaktionskammer (5) des Biochip-Trägers (1) eine Volumenvergrößerungskammer (37) zugeordnet ist.

40. Biochip-Träger nach einem der vorhergehenden Ansprüche, wobei die Volumenvergrößerungskammer (37) durch Seitenwände (29) gebildet wird.

41. Biochip-Träger nach einem der vorhergehenden Ansprüche, wobei die durch die Seitenwände (29) gebildete Volumenvergrößerungskammer (37) Bestandteil einer Volumenvergrößerungsvorrichtung (35) ist.

42. Biochip-Träger nach Anspruch 41, wobei die Volumenvergrößerungsvorrichtung (35) ein Rahmenteil (41) und mindestens eine durch die Seitenwände (29) gebildete Volumenvergrößerungskammer (37) umfasst.

43. Biochip-Träger nach einem der Ansprüche 41 oder 42, wobei die Volumenvergrößerungsvorrichtung (35) in Form eines Streifens oder einer Matrix ausgebildet ist.

44. Biochip-Träger nach einem der vorhergehenden Ansprüche, wobei die Höhe (Hᵥ) der Seitenwände (39) der Volumenvergrößerungskammer (37) größer als die Höhe (H) der Seitenwände (9) der Reaktionskammer (5) ist.

45. Biochip-Träger nach einem der Ansprüche 41 bis 43, wobei die Volumenvergrößerungsvorrichtung (35) so auf den Biochip-Träger (1) aufbringbar ist, dass eine flüssigkeitsdichte Verbindung zwischen den Seitenwänden (9) der Reaktionskammer (5) und den Seitenwänden (39) der Volumenvergrößerungskammer (37) gebildet wird.

46. Biochip-Träger nach einem der Ansprüche 41 bis 43 oder 45, wobei zwischen Biochip-Träger (1) und der reversibel zuordenbaren Volumenvergrößerungsvorrichtung (35) eine Dichtungsmatte (43) angebracht ist.

47. Biochip-Träger nach einem der vorhergehenden Ansprüche, wobei jede Volumenvergrößerungskammer (37) verschließbar ist, insbesondere mittels eines der Volumenvergrößerungskammer (37) zugeordneten Deckels (45).

48. Kit umfassend einen Biochip-Träger (1) nach einem der Ansprüche 1 bis 38 und eine Mikrotiterplatte (100).

49. Kit nach Anspruch 48, umfassend ferner eine Dichtungsmatte (43).

## Claims

1. A biochip platform comprising a platform plate (3) having at least one three-dimensional reaction chamber (5), in which the reaction chamber (5) is formed by a bottom (7) and the volume of the reaction chamber (5), which is open in the upward direction and which is laterally enclosed by side walls (9), where the ratio between the surface area (F) of the bottom (7) and the height (H) of the side walls (9) is greater than or equal to 30, preferably greater than 50, and in which the bottom (7) and/or at least one side wall (9) of at least one reaction chamber (5) is designed as a binding matrix with a functional group, which makes it possible to bind a natural or synthetic molecule, in particular a biologically active molecule, and wherein a volume-enlarging chamber (37) is associated reversibly with the at least one reaction chamber (5), whereby the volume-enlarging chamber (37) can be placed onto the biochip-platform (1) in such a way that a liquid-tight connection is formed between the side walls (9) of the reaction chamber (5) and the side walls (39) of the volume-enlarging chamber (37).

2. The biochip platform of Claim 1, wherein the upper edge (11) of the at least one reaction chamber (5) represents the highest part of the biochip platform (1).

3. The biochip platform of Claim 1 or 2, wherein the upper edge (11) of the at least one reaction chamber (5) lies in a plane above the upper edge (13) of the platform plate (3).

4. The biochip platform of Claim 3, wherein the platform plate (3) forms the bottom (7) of the at least one reaction chamber (5) and the side walls (9) of the reaction chamber (5) are embodied as raised areas on the platform plate (3).

5. The biochip platform of one of Claims 1 to 4, wherein the at least one reaction chamber (5) is disposed on a base (29).

6. The biochip platform of one of Claims 1 to 5, wherein the base (29) has as its contour the shape of the contour of the reaction chamber (5).

7. The biochip platform of one of Claims 1 to 6, wherein the upper surface (31) of the base (29) represents the bottom (7) of the reaction chamber (5).

8. The biochip platform of one of Claims 1 to 7, wherein the side walls (33) of the base (29) lie in a plane with the side walls (9) of the reaction chamber (5).

9. The biochip platform of one of Claims 1 to 8, wherein the side walls (9) of the reaction chamber (5) are embodied as raised areas on the base (29).

10. The biochip platform of Claim 1 or 2, wherein the upper edge (11) of the at least one reaction chamber (5) and the upper edge (13) of the platform plate (3) lie in the same plane.

11. The biochip platform of Claim 10, wherein the at least one reaction chamber (5) is disposed as a depression within and/or below the platform plate (3).

12. The biochip platform of one of Claims 1 to 11, wherein the bottom (7) of the reaction chamber (5), of which at least one is present, seen in the top view has the shape of a circle, rectangle, square, hexagon, polygon, or an ellipse.

13. The biochip platform of one of Claims 1 to 12, wherein the platform plate (3) has dimensions that permit the biochip platform (1) to be inserted into a microtiter plate having dimensions in accordance with the SBS (Society of Bimolecular Screening) standard.

14. The biochip platform of Claim 13, wherein the biochip platform (1) has 1, 2, 4, 8, 12 or in each case integer multiples of 8 or 12 reaction chambers (5).

15. The biochip platform of one of Claims 1 to 13, wherein the platform plate (3) is embodied as a strip.

16. The biochip platform of Claim 15, wherein the platform plate (3) has the dimensions of a standard object holder.

17. The biochip platform of one of the above claims, wherein the platform plate (3) is embodied as a strip having four or, in each case, integer multiples thereof of reaction chambers (5) spaced at a distance one after another.

18. The biochip platform of one of the above claims, wherein the at least one reaction chamber (5) has a circular bottom (7) with a diameter of 6 mm and a side wall height (H) of 0.5 mm.

19. The biochip platform of one of the above claims, wherein the at least one reaction chamber (5) has a square bottom with a side length of 6 mm and a side wall height (H) of 0.5 mm.

20. The biochip platform of one of Claims 1 to 19, wherein the platform plate (3) and the at least one reaction chamber (5) are made of plastic.

21. The biochip platform of Claim 20, wherein the plastic is styrene maleic acid anhydride copolymer (SMA), cycloolefinic copolymer (COC), cycloolefinic polymer (COP), acrylobutadiene styrene, polyamide, polycarbonate, polyester, polymethylmethacrylate, polypropylene, polystyrene, or styrene acrylonitrile.

22. The biochip platform of one of Claims 1 to 21, wherein the bottom (7) of the at least one reaction chamber (5) is made of a polymer, glass, membrane, or hybrid thereof that has on its surface at least one functional group that can bind a biologically active molecule.

23. The biochip platform of Claim 22, wherein the functional group is applied to the polymer surface using a chemical functionalization process.

24. The biochip platform of Claim 23, whereby the polymer of the bottom is a polymer that has aromatic substituents and on whose surface chloromethyl groups are deposited.

25. The biochip platform of Claim 24, wherein the polymer is polystyrene.

26. The biochip platform of Claim 24 or 25, wherein the chloromethyl group of the polymer is substituted by N- or O-nucleophiles.

27. The biochip platform of Claim 26, wherein the chloromethyl group is substituted by an aldehyde group.

28. The biochip platform of Claim 26, wherein the chloromethyl group is substituted by a carboxylic acid.

29. The biochip platform of Claim 23, wherein the polymer of the bottom is a saturated polymer whose surface is chlorinated.

30. The biochip platform of Claim 29, wherein the chlorinated polymer is a chlorinated cycloolefin copolymer, specifically chlorinated TOPAS.

31. The biochip platform of Claim 22, wherein the functional group is applied to the polymer surface using a plasma functionalization process.

32. The biochip platform of Claim 31, wherein the surface has carboxylic acid, aldehyde/ketone, amine, epoxy, and/or halogen functions.

33. The biochip platform of Claim 22, wherein the functional group is applied to the polymer surface using a photo-induced surface functionalization.

34. The biochip platform of Claim 33, wherein the functional group is applied to the polymer surface by reacting anthraquinone with a donor followed by photochemical fixing.

35. The biochip platform of Claim 33, wherein the functional group is applied to the polymer surface by photochemical grafting.

36. The biochip platform of Claim 22, wherein the functional group is applied to the polymer surface using graft polymerization.

37. The biochip platform of one of Claims 22 to 36, wherein a biologically active molecule is bound to the bottom (7) of the reaction chamber (5).

38. The biochip platform of Claim 37, wherein the biologically active molecule is a protein, a nucleic acid, or a PNA molecule.

39. The biochip platform of one of the above claims, wherein each reaction chamber (5) of the biochip platform (1) is associated with a volume-enlarging chamber (37).

40. The biochip platform of one of the above claims, wherein the volume-enlarging chamber (37) is formed by side walls (29).

41. The biochip platform of one of the above claims, wherein the volume-enlarging chamber (37) formed by the side walls (29) is part of a volume-enlarging device (35).

42. The biochip platform of claim 41, wherein the volume-enlarging device (35) comprises a frame part (41) and at least one volume-enlarging chamber (37) formed by the side walls (29).

43. The biochip platform of one of claims 41 or 42, wherein the volume-enlarging device (35) is embodied in the form of a strip or a matrix.

44. The biochip platform of one of the above claims, wherein the height (Hᵥ) of the side walls (39) of the volume-enlarging chamber (37) is greater than the height (H) of the side walls (9) of the reaction chamber (5).

45. The biochip platform of one of claims 41 to 43, wherein the volume-enlarging device (35) can be placed onto the biochip platform (1) in such a way that a liquid-tight connection is formed between the side walls (9) of the reaction chamber (5) and the side walls (39) of the volume-enlarging chamber (37).

46. The biochip platform of one of claims 41 to 43 or 45, wherein a sealing mat (43) is disposed between the biochip platform (1) and the reversibly associable volume-enlarging device (35).

47. The biochip platform of one of the above claims, wherein each volume-enlarging chamber (37) can be closed off, in particular by means of a cover (45) associated with the volume-enlarging chamber (37).

48. A kit comprising a biochip platform (1) as recited in one of Claims 1 to 38 and a microtiter plate (100).

49. A kit of claim 48, also comprising a sealing mat (43).

## Revendications

1. Support de biopuce comprenant une plaque de support (3) avec au moins une chambre réactionnelle tridimensionnelle (5), dans lequel la chambre réactionnelle (5) est formée par une partie de fond (7) et des parois latérales (9) comprenant de côté le volume ouvert vers le haut de la chambre réactionnelle (5), dans lequel le rapport entre la surface (F) de la partie de fond (7) et la hauteur (H) des parois latérales (9) est supérieur ou égal à 30, de préférence supérieur à 50, et dans lequel la partie de fond (7) et/ou au moins une paroi latérale (9) de l'au moins une chambre réactionnelle (5) est réalisée en tant que matrice de liaison avec un groupe fonctionnel qui permet la liaison d'une molécule naturelle ou synthétique, notamment d'une molécule biologiquement active, et dans lequel une chambre d'agrandissement de volume (37) est associée de manière réversible à l'au moins une chambre réactionnelle (5), dans lequel la chambre d'agrandissement de volume (37) peut être appliquée sur le support de biopuce (1) de telle sorte qu'une connexion étanche aux fluides entre les parois latérales (9) de la chambre réactionnelle (5) et les parois latérales (39) de la chambre d'agrandissement de volume (37) est formée.

2. Support de biopuce selon la revendication 1, dans lequel le bord supérieur (11) de l'au moins une chambre réactionnelle (5) représente la partie la plus haute du support de biopuce (1).

3. Support de biopuce selon la revendication 1 ou 2, dans lequel le bord supérieur (11) de l'au moins une chambre réactionnelle (5) se situe dans un plan au-dessus du bord supérieur (13) de la plaque de support (3).

4. Support de biopuce selon la revendication 3, dans lequel la plaque de support (3) forme la partie de fond (7) de l'au moins une chambre réactionnelle (5) et les parois latérales (9) de la chambre réactionnelle (5) sont réalisées en tant que rehaussements sur la plaque de support (3).

5. Support de biopuce selon l'une quelconque des revendications 1 à 4, dans lequel l'au moins une chambre réactionnelle (5) est disposée sur un socle (29).

6. Support de biopuce selon l'une quelconque des revendications 1 à 5, dans lequel le socle (29) présente en plan la forme du plan de la chambre réactionnelle (5).

7. Support de biopuce selon l'une quelconque des revendications 1 à 6, dans lequel la surface supérieure (31) du socle (29) représente la partie de fond (7) de la chambre réactionnelle (5).

8. Support de biopuce selon l'une quelconque des revendications 1 à 7, dans lequel les parois latérales (33) du socle (29) se situent dans un plan avec les parois latérales (9) de la chambre réactionnelle (5).

9. Support de biopuce selon l'une quelconque des revendications 1 à 8, dans lequel les parois latérales (9) de la chambre réactionnelle (5) sont réalisées en tant que rehaussements sur le socle (29).

10. Support de biopuce selon la revendication 1 ou 2, dans lequel le bord supérieur (11) de l'au moins une chambre réactionnelle (5) et le bord supérieur (13) de la plaque de support (3) se situent dans le même plan.

11. Support de biopuce selon la revendication 10, dans lequel l'au moins une chambre réactionnelle (5) est disposée au sein de et/ou en dessous de la plaque de support (3) en tant que cavité.

12. Support de biopuce selon l'une quelconque des revendications 1 à 11, dans lequel la partie de fond (7) de l'au moins une chambre réactionnelle (5) présente, vue en vue de dessus, la forme d'un cercle, rectangle, carré, hexagone, polygone ou d'une ellipse.

13. Support de biopuce selon l'une quelconque des revendications 1 à 12, dans lequel la plaque de support (3) présente des dimensions qui permettent l'insertion du support de biopuce (1) dans une plaque de microtitration avec des dimensions selon la norme SBS (Society of Biomolecular Screening).

14. Support de biopuce selon la revendication 13, dans lequel le support de biopuce (1) présente 1, 2, 4, 8, 12 ou des multiples à chaque fois entiers de 8 ou 12 chambres réactionnelles (5).

15. Support de biopuce selon l'une quelconque des revendications 1 à 13, dans lequel la plaque de support (3) est réalisée en tant que bande.

16. Support de biopuce selon la revendication 15, dans lequel la plaque de support (3) présente les dimensions d'un porte-objet standard.

17. Support de biopuce selon l'une quelconque des revendications précédentes, dans lequel la plaque de support (3) est réalisée en tant que bande avec 4 ou des multiples à chaque fois entiers de 4 chambres réactionnelles (5) écartées, disposées les unes derrière les autres.

18. Support de biopuce selon l'une quelconque des revendications précédentes, dans lequel l'au moins une chambre réactionnelle (5) présente une partie de fond circulaire (7) avec un diamètre de 6 mm et une hauteur de paroi latérale (H) de 0,5 mm.

19. Support de biopuce selon l'une quelconque des revendications précédentes, dans lequel l'au moins une chambre réactionnelle (5) présente une partie de fond carrée avec une longueur de côté de 6 mm et une hauteur de paroi latérale (H) de 0,5 mm.

20. Support de biopuce selon l'une quelconque des revendications 1 à 19, dans lequel la plaque de support (3) et l'au moins une chambre réactionnelle (5) se composent de plastique.

21. Support de biopuce selon la revendication 20, dans lequel il s'agit pour le plastique d'un copolymère de styrène-anhydride d'acide maléique (SMA), copolymère de cyclooléfine (COC), polymère de cyclooléfine (COP), butadiènestyrène acrylique, polyamide, polycarbonate, polyester, polyméthylméthacrylate, polypropylène, polystyrène ou acrylonitrile de styrène.

22. Support de biopuce selon l'une quelconque des revendications 1 à 21, dans lequel la partie de fond (7) de l'au moins une chambre réactionnelle (5) se compose d'un polymère, de verre, d'une membrane ou d'un hybride de celui-ci qui présente au moins un groupe fonctionnel à la surface, lequel peut se fixer à une molécule biologiquement active.

23. Support de biopuce selon la revendication 22, dans lequel le groupe fonctionnel est appliqué sur la surface du polymère en utilisant un procédé de fonctionnalisation chimique.

24. Support de biopuce selon la revendication 23, dans lequel le polymère de la partie de fond est un polymère avec des résidus aromatiques à la surface duquel des groupes chlorométhyle sont fixés.

25. Support de biopuce selon la revendication 24, dans lequel le polymère est du polystyrène.

26. Support de biopuce selon la revendication 24 ou 25, dans lequel le groupe chlorométhyle du polymère est substitué par des N- ou O-nucléophiles.

27. Support de biopuce selon la revendication 26, dans lequel le groupe chlorométhyle est substitué par un groupe aldéhyde.

28. Support de biopuce selon la revendication 26, dans lequel le groupe chlorométhyle est substitué par un acide carboxylique.

29. Support de biopuce selon la revendication 23, dans lequel le polymère de la partie de fond est un polymère saturé dont la surface est chlorée.

30. Support de biopuce selon la revendication 29, dans lequel le polymère chloré est un copolymère de cyclooléfine chloré, notamment TOPAS chloré.

31. Support de biopuce selon la revendication 22, dans lequel le groupe fonctionnel est appliqué sur la surface du polymère en utilisant un procédé de fonctionnalisation au plasma.

32. Support de biopuce selon la revendication 31, dans lequel la surface présente des fonctions acide carboxylique, aldéhyde/cétone, amine, époxy et/ou halogène.

33. Support de biopuce selon la revendication 22, dans lequel le groupe fonctionnel est appliqué sur la surface du polymère en utilisant une fonctionnalisation superficielle photo-induite.

34. Support de biopuce selon la revendication 33, dans lequel le groupe fonctionnel est appliqué sur la surface du polymère par conversion d'anthraquinone avec un donneur, puis fixation photochimique.

35. Support de biopuce selon la revendication 33, dans lequel le groupe fonctionnel est appliqué sur la surface du polymère par greffe photochimique.

36. Support de biopuce selon la revendication 22, dans lequel le groupe fonctionnel est appliqué sur la surface du polymère en utilisant une polymérisation par greffage.

37. Support de biopuce selon l'une quelconque des revendications 22 à 36, dans lequel une molécule biologiquement active est fixée sur la partie de fond (7) de la chambre réactionnelle (5).

38. Support de biopuce selon la revendication 37, dans lequel la molécule biologiquement active est une protéine, un acide nucléique ou une molécule d'acide peptidonucléique.

39. Support de biopuce selon l'une quelconque des revendications précédentes, dans lequel une chambre d'agrandissement de volume (37) est associée à chaque chambre réactionnelle (5) du support de biopuce (1).

40. Support de biopuce selon l'une quelconque des revendications précédentes, dans lequel la chambre d'agrandissement de volume (37) est formée par des parois latérales (29).

41. Support de biopuce selon l'une quelconque des revendications précédentes, dans lequel la chambre d'agrandissement de volume (37) formée par les parois latérales (29) fait partie d'un dispositif d'agrandissement de volume (35).

42. Support de biopuce selon la revendication 41, dans lequel le dispositif d'agrandissement de volume (35) comprend une partie de cadre (41) et au moins une chambre d'agrandissement de volume (37) formée par les parois latérales (29).

43. Support de biopuce selon l'une quelconque des revendications 41 ou 42, dans lequel le dispositif d'agrandissement de volume (35) est réalisé sous la forme d'une bande ou d'une matrice.

44. Support de biopuce selon l'une quelconque des revendications précédentes, dans lequel la hauteur (Hᵥ) des parois latérales (39) de la chambre d'agrandissement de volume (37) est supérieure à la hauteur (H) des parois latérales (9) de la chambre réactionnelle (5).

45. Support de biopuce selon l'une quelconque des revendications 41 à 43, dans lequel le dispositif d'agrandissement de volume (35) peut être appliqué sur le support de biopuce (1) de telle sorte qu'une connexion étanche aux fluides entre les parois latérales (9) de la chambre réactionnelle (5) et les parois latérales (39) de la chambre d'agrandissement de volume (37) est formée.

46. Support de biopuce selon l'une quelconque des revendications 41 à 43 ou 45, dans lequel un tapis d'étanchéité (43) est monté entre le support de biopuce (1) et le dispositif d'agrandissement de volume (35) pouvant être associé de manière réversible.

47. Support de biopuce selon l'une quelconque des revendications précédentes, dans lequel chaque chambre d'agrandissement de volume (37) peut être fermée, notamment au moyen d'un couvercle (45) associé à la chambre d'agrandissement de volume (37).

48. Kit comprenant un support de biopuce (1) selon l'une quelconque des revendications 1 à 38 et une plaque de microtitration (100).

49. Kit selon la revendication 48, comprenant en outre un tapis d'étanchéité (43).
